# EUROPÄISCHE PATENTANMELDUNG

(11) **EP 3 666 317 A1**
(43) Veröffentlichungstag der Anmeldung: **17.06.2020**
(21) Anmeldenummer: 19207111.6
(22) Anmeldetag: 24.05.2016
(51) Int. Cl.: A61M 11/06, A61B 5/087

(54) **INHALIERSYSTEM**

(30) Priorität: 26.05.2015 EP 15169181
(62) Teilanmeldung aus: 16171017.3
(71) Anmelder: PARI GmbH Spezialisten für effektive Inhalation, 82319 Starnberg (DE)
(72) Erfinder: Gramann, Jens, 82166 Gräfelfing (DE); Jelovac, Emir, 80639 München (DE); Selzer, Titus, 80686 München (DE); Buchmann, Nicolas, 82008 Unterhaching (DE)
(74) Vertreter: Hoffmann Eitle

(57) **Zusammenfassung**

Inhaliersystem mit einer Inhaliervorrichtung und einem Flussmesser (12), der einen Strömungsraum (15), einen Widerstandskörper (20) und einen Flussanzeiger (21) aufweist, wobei der Strömungsraum (15) eine Einlassöffnung (17), die wirksam mit der Umgebung verbindbar ist, eine Auslassöffnung (18), die wirksam mit einem Innenraum der Inhaliervorrichtung verbindbar ist, und eine Flusswiderstandseinrichtung aufweist, das Inhaliersystem eingerichtet ist, eine Zuluft durch die Einlassöffnung (17) in den Strömungsraum (15), durch die Auslassöffnung (18) aus dem Strömungsraum (15) heraus und in den Innenraum der Inhaliervorrichtung zu leiten, der Widerstandskörper (20) eingerichtet ist, in dem Strömungsraum (15) verschiedene Positionen einnehmen zu können, und der Flussanzeiger (21) eingerichtet ist, eine Position des Widerstandskörpers (20) in dem Strömungsraum (15) anzuzeigen.

## Beschreibung

Die Erfindung betrifft ein Inhaliersystem mit einer Inhaliervorrichtung.

Im Stand der Technik sind Inhaliersysteme bekannt.

WO 2005/042075 A1 beschreibt eine Inhalationstherapievorrichtung mit einer Verneblerkammer und einem Aerosolerzeuger, der derart angeordnet ist, dass er ein Aerosol in die Verneblerkammer abgibt, und ein Düsenelement umfasst. Diese Inhalationstherapievorrichtung gibt jedoch keinen Hinweis darauf, ob der erzeugte Einatemfluss für eine Inhalation geeignet ist.

DE 197 34 022 C2 beschreibt ein Inhalationstherapiegerät mit einem Ventil zur Begrenzung des Inspirationsflusses. Dieses Inhalationstherapiegerät gibt einen Hinweis, wenn der Einatemfluss zu groß ist. Es gibt jedoch keinen Hinweis darauf, welcher Einatemfluss anzustreben ist, und wie sich der erzeugte Einatemfluss dazu verhält.

Die Aufgabe der Erfindung besteht darin, ein Inhaliersystem bereitzustellen, das einen Hinweis darauf gibt, ob ein erzeugter Einatemfluss für eine Inhalation geeignet ist.

Die Aufgabe wird gelöst durch ein Inhaliersystem mit einer Inhaliervorrichtung und einem Flussmesser, der einen Strömungsraum, einen Widerstandskörper und einen Flussanzeiger aufweist, wobei der Strömungsraum eine Einlassöffnung, die wirksam mit der Umgebung verbindbar ist, eine Auslassöffnung, die wirksam mit einem Innenraum der Inhaliervorrichtung verbindbar ist, und eine Flusswiderstandseinrichtung aufweist, das Inhaliersystem eingerichtet ist, eine Zuluft durch die Einlassöffnung in den Strömungsraum, durch die Auslassöffnung aus dem Strömungsraum heraus und in den Innenraum der Inhaliervorrichtung zu leiten, der Widerstandskörper eingerichtet ist, in dem Strömungsraum verschiedene Positionen einnehmen zu können, und der Flussanzeiger eingerichtet ist, eine Position des Widerstandskörpers in dem Strömungsraum anzuzeigen.

Vorzugsweise ist die Inhaliervorrichtung eine Aerosolerzeugungsvorrichtung. Eine Aerosolerzeugungsvorrichtung weist bevorzugt einen Vernebler, einen Zerstäuber, einen Befeuchter, einen Druckluftvernebler, einen Luftzerstäuber, einen elektronischen Vernebler, einen Ultraschallvernebler, einen elektrohydrodynamischen Vernebler, einen elektrostatischen Vernebler, einen Membranvernebler, einen Vernebler mit einer vibrierenden Membran, einen elektronischen Vernebler mit einer vibrierenden Membran, einen Mesh-Vernebler, einen Düsenvernebler, einen Inhalator (MDI), einen Pulverzerstäuber (DPI) oder eine Kombination davon auf. Der Inhalator weist in einer Ausführungsform einen unter Druck stehenden Kanister mit einem Medikament und einem Treibgas auf. Zweckmäßigerweise ist der Kanister mit einem von Hand bedienbaren Aktuator verbunden. Es ist vorteilhaft, wenn der Inhalator eingerichtet ist, bei der Aktivierung eine bestimmte Medikamentenmenge in Aerosolform abzugeben. In einer Ausführungsform ist die Aerosolerzeugungsvorrichtung für den Einsatz mit Beatmungsvorrichtungen eingerichtet.

Es ist vorteilhaft, wenn die Inhaliervorrichtung eine Vorrichtung zur Bereitstellung von Aerosolen ist. Die Vorrichtung zur Bereitstellung von Aerosolen weist bevorzugt eine Inhalierhilfe, einen Spacer oder eine Chamber auf. Vorrichtungen zur Bereitstellung von Aerosolen sind vorzugsweise Vorrichtungen, die zur Verwendung mit Inhalatoren (MDIs) vorgesehen sind. Sie stellen Medikamentenräume bereit, die dazu eingerichtet sind, Aerosol bevorzugt aus Inhalatoren aufzunehmen, so dass es von Benutzern daraus abgeatmet werden kann. Spacer weisen keine Ein- und Ausatemventile auf, so dass ein Benutzer seine Atmung so koordinieren sollte, dass er nicht in den Spacer ausatmet. Chambers oder Holding Chambers weisen Aus- und bevorzugt auch Einatemventile auf. So kann erreicht werden, dass ein Ausatemstrom nicht in den Raum, in dem sich das Aerosol befindet, geleitet wird. Es kann erreicht werden, dass ein Medikament nur beim Einatmen aus dem Medikamentenraum heraus gelangen kann.

Aerosole sind Gemische aus festen oder flüssigen Schwebeteilchen und einem Gas.

Aerosole sind bevorzugt zur Applikation auf oder in Teile des menschlichen oder tierischen Körpers, wie Haut, Körperhöhlen, Körperöffnungen, Nase, Nasennebenhöhlen, Kieferhöhle, Stirnhöhle, Keilbeinhöhle, Siebbeinzellen, Rachen, Kehlkopf, Luftröhre, Lunge, Stammbronchus, Bronchien, Bronchiolen, Lungenbläschen, Gelenke oder Bauchraum vorgesehen. Aerosole können eingesetzt werden, um Krankheiten von Menschen und Tieren zu diagnostizieren, vorzubeugen, zu therapieren oder um Menschen oder Tiere gegen Krankheiten zu immunisieren.

Ein Flussmesser ist eine Einrichtung, die dazu eingerichtet ist eine Eigenschaft eines Flusses festzustellen. Die Eigenschaft ist zweckmäßigerweise eine Geschwindigkeit, eine Richtung, ein Volumen, ein Volumen je Zeiteinheit oder eine Masse. Der Flussmesser ist vorzugsweise geeignet, Werte, das Überschreiten von Grenzwerten oder das Vorliegen von bestimmten Bereichen festzustellen. In einer Ausführungsform ist der Flussmesser dazu eingerichtet, Einatemluft derart durch den Strömungsraum zu führen, dass sie darin aufwärts strömt. Vorzugsweise ist der Flussmesser eingerichtet, die Einatemluft über einen Bogen zu der Inhaliervorrichtung umzulenken. Zweckmäßigerweise ist der Flussmesser eingerichtet, der Inhaliervorrichtung Einatemluft zuzuführen.

Der Strömungsraum weist zweckmäßigerweise einen Hohlraum auf. Der Hohlraum weist vorzugsweise eine Konusform, eine Hohlzylinderform, eine Hohlkegelform oder einen beliebigen Querschnitt auf. Es ist zweckmäßig, wenn der Hohlraum eine längliche Form aufweist. In einer Ausführungsform weist der Hohlraum einen kontinuierlich zunehmenden Querschnitt auf. Der Querschnitt nimmt zweckmäßigerweise in eine Richtung zu, die dazu vorgesehen ist, im Betrieb nach oben, entgegen der Schwerkraft der Erde, zu sein. In einer Ausführungsform nimmt der Querschnitt in eine Richtung schräg nach oben zu. Der Strömungsraum weist vorzugsweise eine Wand aus Kunststoff, Glas oder Metall auf. In einer Ausführungsform weist die Wand einen durchsichtigen Bereich auf oder ist überall durchsichtig. Besonders bevorzugt weist die Wand einen durchsichtigen Kunststoff auf. Es ist besonders zweckmäßig, wenn die Wand ein Spritzgussteil aufweist. In einer Ausführungsform weist die Wand ein Rohr auf. Es ist vorteilhaft, wenn die Wand einen rechteckigen oder vieleckigen Querschnitt aufweist. In einer Ausführungsform ist der Strömungsraum in ein anderes Bauteil des Inhaliersystems integriert. Vorzugsweise ist der Strömungsraum in einen Kamin eines Düsenverneblers integriert. Der Kamin ist ein Bauteil, das eingerichtet ist, Umgebungsluft zu einer Düse zu leiten. Es ist bevorzugt, wenn die Umgebungsluft durch einen Einatemunterdruck zu der Düse leitbar ist. Zweckmäßigerweise ist zusätzlich ein unter Druck stehendes Gas, vorzugsweise von einem Kompressor oder aus einer Gasflasche, zu der Düse leitbar.

In einer Ausführungsform weist der Widerstandskörper eine Kugel, einen Kegel, einen Kegelstumpf, einen Zylinder oder eine Kombination daraus auf. Vorzugsweise ist der Widerstandskörper rotationssymmetrisch.

Strömungsraum und Widerstandskörper sind in einer Ausführungsform dazu ausgelegt, dass der Widerstandskörper in dem Strömungsraum beeinflusst von der Schwerkraft und einem Gas, das dazu vorgesehen ist, durch den Strömungsraum strömen zu können, unterschiedliche Positionen einnehmen kann. Der Widerstandskörper ist zweckmäßigerweise dazu vorgesehen, mit dem Einatemfluss eines Patienten angehoben zu werden. Der Widerstandskörper ist bevorzugt dazu eingerichtet, abhängig vom Einatemfluss auf verschiedenen Höhen zu schweben.

Ein Flussanzeiger ist eine Einrichtung, die dazu geeignet ist, eine Eigenschaft eines Flusses anzuzeigen. Es ist vorteilhaft, wenn der Flussanzeiger eingerichtet ist, die Eigenschaft des Flusses optisch oder akustisch anzuzeigen. In einer Ausführungsform ist der Flussanzeiger eingerichtet, die Eigenschaft des Flusses diskret anzuzeigen. Bevorzugt ist es, wenn der Flussanzeiger eingerichtet ist, die Eigenschaft des Flusses kontinuierlich anzuzeigen. Der Flussanzeiger weist in einer Ausführungsform einen durchsichtigen Bereich auf, durch den der Widerstandskörper zumindest in einer Position sichtbar ist. Es ist sinnvoll, wenn der Widerstandskörper in einer Position sichtbar ist, die einen optimalen Einatemstrom kennzeichnet. Es ist vorteilhaft, wenn der Flussanzeiger eingerichtet ist, die Position des Widerstandskörpers über ein Magnetsystem auf eine mechanische oder elektrische Anzeige zu übertragen. Durch eine Anbringung des Flussanzeigers in einem Bereich, der im Betriebszustand im Gesichtsfeld des Patienten ist, kann der Patient selbst beim Inhalieren ein Feedback zu dem erzielten Einatemfluss erhalten. Der Patient ist derjenige, der das Inhaliersystem verwendet, um auf oder in Teile seines Körpers ein Fluid aus dem Inhaliersystem zuzuführen.

Die Flusswiderstandseinrichtung weist vorzugsweise einen in Öffnungsrichtung zunehmenden Querschnitt des Strömungsraums auf. Der Querschnitt nimmt vorzugsweise in Strömungsrichtung zu. In einer Ausführungsform nimmt der Querschnitt in Richtung Inhaliervorrichtung zu. Es ist zweckmäßig, wenn der Querschnitt in Richtung Auslassöffnung zunimmt. Es ist bevorzugt, wenn der Querschnitt in eine Richtung zunimmt, die im Betrieb des Inhaliersystems entgegen der Schwerkraft der Erde zeigt. In einer Ausführungsform nimmt der Querschnitt in eine Richtung zu, die im Betrieb des Inhaliersystems schräg nach oben zeigt. Auf diese Weise kann der Widerstandskörper durch eine Strömung entgegen der Schwerkraft bewegt werden.

In einer Ausführungsform weist die Flusswiderstandseinrichtung eine Feder oder einen Magneten auf. Zweckmäßigerweise ist die Feder oder der Magnet eingerichtet, eine Kraft auf den Widerstandskörper ausüben zu können. Es ist bevorzugt, wenn die Feder oder der Magnet eingerichtet ist, mit zunehmendem Fluss durch den Strömungsraum eine größere Kraft auf den Widerstandskörper auszuüben. Es ist zweckmäßig, wenn die Flusswiderstandseinrichtung derart eingerichtet ist, dass im Betrieb der Luftwiderstand an dem Widerstandskörper gegen die Feder oder den Magneten arbeitet. Zweckmäßigerweise ist der Widerstandskörper eingerichtet, sich mit zunehmendem Durchfluss durch den Strömungsraum weiter in Öffnungsrichtung zu bewegen und eine stärkere Kraft auf die Feder auszuüben. Die Bewegung des Widerstandskörpers kann durch Auslegung der Federkennlinie sinnvoll abgestimmt werden. In einer Ausführungsform ist der Querschnitt des Strömungsraums in allen Bereichen, die dazu vorgesehen sind, den Widerstandskörper aufzunehmen, gleich groß. Vorzugsweise ist der in eine Öffnungsrichtung zunehmende Querschnitt durch eine konusförmige Ausgestaltung oder durch einen sich in Öffnungsrichtung gleichmäßig in alle Richtungen aufweitenden Querschnitt realisiert. Der Querschnitt kann quadratisch, rechteckig, dreieckig, oval oder unregelmäßig ausgebildet sein. In einer Ausführungsform ist der in eine Öffnungsrichtung zunehmende Querschnitt durch die Kombination eines in Öffnungsrichtung konstanten Querschnittsbereichs mit einem in Öffnungsrichtung zunehmenden Querschnittsbereich ausgebildet. Bevorzugt weisen dabei sowohl ein Rand des konstanten Querschnittsbereichs als auch ein Rand des in Öffnungsrichtung zunehmenden Querschnittsbereichs ein Kreissegment auf. Zweckmäßigerweise ist die Öffnungsrichtung im Betrieb des Inhaliersystems entgegen der Schwerkraft der Erde gerichtet.

Zuluft weist vorzugsweise Umgebungsluft auf. In einer Ausführungsform ist Zuluft dazu vorgesehen, zusätzlich zu einem anderen Gas einem Innenraum einer Inhaliervorrichtung zugeführt zu werden. Zweckmäßigerweise ist das andere Gas ein Gas aus einem Inhalator oder ein Gas aus einer Düse, die zur Aerosolerzeugung vorgesehen ist. In einer Ausführungsform weist das andere Gas ebenfalls Umgebungsluft auf und ist dazu vorgesehen, direkt aus der Umgebung in den Innenraum der Inhaliervorrichtung geführt zu werden. Zweckmäßigerweise ist die Zuluft dazu vorgesehen, eingeatmet zu werden.

Die Einlassöffnung ist vorzugsweise derart angeordnet, dass sie im Betriebszustand von dem Anwender weg gerichtet ist. So kann verhindert werden, dass Ausatemluft in die Messeinrichtung gelangt und diese beispielsweise mit Aerosolresten kontaminiert wird. Zweckmäßigerweise ist dies durch die Verwendung eines Verlängerungsstücks, vorzugsweise durch die Verwendung eines Schlauchs verwirklicht.

Ein Innenraum der Inhaliervorrichtung ist vorzugsweise dazu eingerichtet, Fluide, wie Flüssigkeiten, Gase oder Aerosole oder mehrere davon aufzunehmen oder zu verändern, wie zu mischen oder weiterzuleiten.

Das Inhaliersystem weist vorzugsweise einen Kunststoff, besonders bevorzugt Polypropylen oder Polyamid auf. In einer Ausführungsform weist das Inhaliersystem einen durchsichtigen Kunststoff auf. Bevorzugt weisen ein oder mehrere Bauteile des Inhaliersystems Kunststoffspritzgussteile auf.

Wenn der Flussanzeiger im Sicht- oder Hörbereich eines Patienten angeordnet ist, kann der Patient besonders effektiv sein Einatem-Manöver beobachten und beeinflussen.

Es ist vorteilhaft, wenn spezifische Flussmesser und Verneblereinsätze geometrisch codiert sind, so dass spezifische Flussmesser nur zusammen mit bestimmten Verneblereinsätzen benutzbar sind. In einer Ausführungsform ist ein spezifischer Flussmesser mit einem bestimmten Verneblereinsatz verbunden. Vorteile sind erzielbar, wenn der spezifische Flussmesser in einen Vernebler integriert ist.

Ein Verneblereinsatz ist ein Einsatz, der in einen Vernebler einbringbar ist. Vorzugsweise weist der Verneblereinsatz einen Filter auf, der eingerichtet ist, Tröpfchen, die einen vorgegebenen Wert übersteigen auszufiltern. Zweckmäßigerweise weist der Filter einen Prallschirm auf, der eingerichtet ist, den Aerosolstrom derart zu leiten, dass Tröpfchen, die eine vorgegebene Größe überschreiten, gegen den Prallschirm prallen und aus dem Aerosol entfernt werden.

Es ist vorteilhaft, wenn der Flussmesser auf unterschiedliche Flussbereiche hin optimiert ist. In einer Ausführungsform ist ein leichter Widerstandskörper vorgesehen, der vorzugsweise für Kinder vorgesehen ist. Es ist zweckmäßig, wenn der Spalt zwischen dem Widerstandskörper und dem Strömungsraumbehälter an einen bestimmten Flussbereich angepasst ist. Vorzugsweise ist diese Anpassung durch bestimmte Durchmesser oder Steigungen des Strömungsraumbehälters und Durchmesser des Widerstandskörpers verwirklicht. Es ist zweckmäßig, wenn die Masse des Widerstandskörpers an den Flussbereich angepasst ist.

In einer Ausgestaltung ist das Inhaliersystem eingerichtet, eine Strömungsrichtung der Zuluft stromabwärts des Strömungsraums zu ändern. Auf diese Weise kann der Flussanzeiger durch einfache konstruktive Maßnahmen im Betrieb im Sichtbereich eines Anwenders, wie dem Gesichtsfeld eines Patienten, angeordnet sein. Der Flussanzeiger kann derart angeordnet sein, dass er sich beim Inhalieren oberhalb der Inhaliervorrichtung befindet, so dass ein Patient den Flussanzeiger beim Inhalieren auf einfache Weise sehen kann. Der Flussanzeiger kann auch in anderen günstigen Bereichen angeordnet sein. Das Inhaliersystem kann dazu eingerichtet sein, die Strömung nach der Durchströmung des Strömungsraums um eine Kurve zu leiten oder die Strömung nach der Durchströmung des Strömungsraums in eine Gegenrichtung zu leiten. So kann eine kompakte Bauweise erreicht werden.

In einer Ausführungsform ist stromabwärts des Strömungsraums eine Vorwärmeinrichtung vorgesehen. Auf diese Weise kann die Zuluft vorgewärmt werden. Durch die Änderung der Strömungsrichtung kann die Vorwärmeinrichtung kompakt und effektiv realisiert werden.

Durch gezielte Richtungsänderungen kann erreicht werden, dass unerwünschte Bestandteile der Zuluft, wie Staub oder Wassertropfen nicht in den Innenraum der Inhaliervorrichtung gelangen.

Es ist zweckmäßig, wenn der Strömungsraum derart ausgestaltet ist, dass der Widerstandskörper in einem Betriebszustand des Inhaliersystems in dem Strömungsraum eingeschlossen ist. So kann erreicht werden, dass der Widerstandskörper auch bei zu großen oder zu kleinen Einatemflüssen in dem Strömungsraum bleibt. Dadurch kann die Funktion des Flussmessers und des Flussanzeigers auch bei einem stark von einem idealen Einatemfluss abweichenden Einatemfluss erhalten werden. Der Widerstandskörper ist eingeschlossen, wenn das Inhaliersystem dazu eingerichtet ist, den Widerstandskörper daran zu hindern, den Strömungsraum zu verlassen. Vorzugsweise ist das Inhaliersystem dazu eingerichtet, den Widerstandskörper daran zu hindern, bei Überschreiten einer maximalen Einatemgeschwindigkeit oder bei Unterschreiten einer minimalen Einatemgeschwindigkeit den Strömungsraum zu verlassen. Zweckmäßigerweise sind die Öffnungen des Strömungsraums so klein, dass der Widerstandskörper nicht hindurchpasst. In einer Ausführungsform ist dies dadurch verwirklicht, dass die Einlassöffnung und die Auslassöffnung einen Querschnitt aufweisen, der kleiner ist als der maximale Querschnitt des Widerstandskörpers. Es ist zweckmäßig, wenn der Strömungsraum außer der Einlassöffnung und der Auslassöffnung keine weiteren Öffnungen aufweist. Um zu verhindern, dass der Widerstandskörper den Strömungsraum durch die Einlassöffnung oder durch die Auslassöffnung verlässt, kann ein Steg oder eine Nase vorgesehen sein. In einer Ausführungsform ist der Steg oder die Nase an der Einlass- oder Auslassöffnung oder an beiden vorgesehen. Der Steg oder die Nase können in einem Bereich, der innerhalb des Strömungsraums benachbart zu der Einlass- oder Auslassöffnung oder beiden liegt, vorgesehen sein.

In einer Ausgestaltung ist der Widerstandskörper permanent in dem Strömungsraum eingeschlossen. So kann verhindert werden, dass der Widerstandskörper versehentlich verschluckt wird. Auch bei einer Demontage kann ein Verlorengehen oder versehentliches Verschlucken des Widerstandskörpers verhindert werden. Der Widerstandskörper ist permanent in dem Strömungsraum eingeschlossen, wenn er nicht zerstörungsfrei aus dem Strömungsraum entfernt werden kann. In einer Ausführungsform ist dies dadurch verwirklicht, dass eine oder mehrere Wände des Strömungsraums einen Käfig oder eine Kapsel bilden. Es ist vorteilhaft, wenn ein Deckel mit einer Wand des Schweberaums form- oder kraftschlüssig verbunden ist. Zweckmäßigerweise weist eine Wand des Strömungsraums eine Schweißstelle, eine Lötstelle, eine Klebestelle, einen Niet oder eine Raste auf. In einer Ausführungsform ist ein Deckel mit einer Wand des Strömungsraums verschweißt.

Damit der Widerstandskörper nicht von spielenden Kindern versehentlich verschluckt oder eingeatmet werden kann, kann vorzugsweise nach der Montage des Widerstandskörpers eine Geometrie in das Steigrohr eingebracht werden, die verhindert, dass der Widerstandskörper aus dem Steigrohr entfernt werden kann. In einer Ausführungsform ist diese Struktur in ein elastisches Element integriert, das einrasten kann. In einer Ausführungsform ist ein Deckel vorgesehen, der nicht demontierbar ausgeführt, vorzugsweise verrastet, verclipst oder verschweißt ist.

Es ist vorteilhaft, wenn der Flussmesser dazu eingerichtet ist, trennbar mit der Inhaliervorrichtung verbunden zu sein. Dies kann die Reinigung erleichtern. Eine trennbare Verbindung ist vorzugsweise durch Verschrauben, einen Klettverschluss, Reibschluss, Formschluss oder eine Konusverbindung realisiert. In einer Ausführungsform ist der Flussmesser über einen Konus auf einen Kamin eines Düsenverneblers aufsteckbar. Vorzugsweise ist die Inhaliervorrichtung auch ohne den Flussmesser funktionsfähig. Dadurch kann der Flussmesser als Zusatzbauteil eingesetzt werden, das entfernt werden kann, wenn es nicht notwendig ist. Dies kann der Fall sein, wenn der Patient gelernt hat, einen geeigneten Einatemfluss zu erzeugen. Zweckmäßigerweise ist das Inhaliersystem derart eingerichtet, dass der Flussmesser entfernbar und ein Einatemventil anbringbar ist. In einer Ausführungsform ist das Inhaliersystem derart ausgebildet, dass ein Einatemventil in dem Bereich anbringbar ist, in dem der Flussmesser anbringbar ist. Es ist vorteilhaft, wenn das Inhaliersystem dazu ausgestaltet ist, gleichzeitig einen Flussmesser und ein davon unabhängig einsetzbares Einatemventil aufzuweisen. Besonders vorteilhaft ist es, wenn das Inhaliersystem ein von dem Flussmesser unabhängiges Einatemventil aufweist. So kann der Flussmesser auf besonders einfache Weise entfernt werden und das Inhaliersystem ohne Flussmesser betrieben werden. Der Flussmesser kann dazu eingerichtet sein, im Bypass betrieben zu werden. In einer Ausführungsform ist Umgebungsluft sowohl durch den Flussmesser als auch durch ein von dem Flussmesser unabhängiges Einatemventil in den Innenraum der Inhaliervorrichtung leitbar. In einer bevorzugten Ausführungsform ist der Flussmesser derart mit der Inhaliervorrichtung verbindbar, dass die Auslassöffnungen des Flussmessers und das Einatemventil derart verbunden sind, dass ein Gas, das aus der Auslassöffnung heraustritt, zu dem Einatemventil leitbar ist. In einer besonders zweckmäßigen Ausführungsform ist der Flussmesser derart mit dem Inhaliersystem verbindbar, dass das Einatemventil bei mit dem Inhaliersystem verbundenem Flussmesser in einer geschlossenen Position gehalten ist.

In einer Ausführungsform weist der Strömungsraum eine Führungsvorrichtung für den Widerstandskörper auf. Auf diese Weise kann ein Verkanten des Widerstandskörpers besser verhindert werden. Die übrige Form des Querschnitts des Strömungsraums kann vergleichsweise unabhängig von der Führungsfunktion gestaltet werden. Vorzugsweise ist eine Führungsrippe im Inneren des Strömungsraums als Führungsvorrichtung vorgesehen. Die Führungsrippe ist zweckmäßigerweise in Längsrichtung angeordnet. In einer Ausführungsform sind mehrere, vorzugsweise drei Führungsrippen vorgesehen. In einer Ausgestaltung weist der Querschnitt des Strömungsraums einen ersten Teilbereich mit einer Führungsvorrichtung auf, der in Öffnungsrichtung weitgehend unveränderlich ist und einen zweiten Teilbereich, der sich in Öffnungsrichtung aufweitet. Die Führungsvorrichtung kann einen Teil eines Hohlzylinders aufweisen.

Es ist vorteilhaft, wenn Widerstandskörper und Strömungsraum eingerichtet sind, bei Überschreiten einer Grenzströmung einen Durchströmungsquerschnitt in dem Strömungsraum zu verkleinern. Dadurch wird der Einatemwiderstand bei Überschreiten der Grenzströmung für den Patienten größer. In einer Ausführungsform sind Widerstandskörper und Strömungsraum eingerichtet, bei Überschreiten der Grenzströmung den Durchströmungsquerschnitt in dem Strömungsraum abzudichten.

Um eine ausreichende Menge Einatemluft durch das Inhaliersystem einatmen zu können, muss der Patient langsamer einatmen. Auf diese Weise kann einem Benutzer eine spezielle Rückmeldung gegeben werden, wenn der Einatemfluss zu hoch ist.

In einer Ausführungsform ist der Querschnitt des Strömungsraums in einem Bereich, in dem sich der Widerstandskörper bei Erreichen der Grenzströmung befindet kleiner als in dem Bereich, in dem sich der Widerstandskörper bei einem optimalen Einatemstrom befindet. Die Querschnittsverringerung kann stufenweise oder kontinuierlich vorgesehen sein, je nachdem, welche Rückmeldung für den Patienten gewünscht ist. In einer Ausführungsform ist der Widerstandskörper eingerichtet, bei Überschreiten der Grenzströmung die Auslassöffnung abzudichten. An der Auslassöffnung kann eine Gummidichtung vorgesehen sein. Durch die Form des Widerstandskörpers oder der Gummidichtung kann die Rückmeldung für den Patienten beeinflusst werden. Eine sich verjüngende Form des Widerstandskörpers an der Seite, die der Auslassöffnung zugewandt ist, kann ein Ansteigen des Einatemwiderstands bis zur Blockade des Einatemstroms erzielen, indem die Auslassöffnung durch den Widerstandskörper mit zunehmendem Unterdruck immer weiter verschlossen wird. Vorzugsweise weist die sich verjüngende Form eine Kegelform oder Kegelstumpfform auf. Eine flache Form des Widerstandskörpers an der Seite, die der Auslassöffnung zugewandt ist, kann ein plötzliches Ansteigen des Einatemwiderstands mit einer schnellen Blockade des Einatemflusses oder eine Blockade des Einatemflusses ohne nennenswertes vorheriges Ansteigen des Einatemwiderstands erzielen. Zweckmäßigerweise ist die flache Form ein plane Fläche oder eine gekrümmte Fläche, wobei die gekrümmte Fläche große Krümmungsradien aufweist.

In einer Ausführungsform sind Strömungsraum und Widerstandskörper eingerichtet, bei Unterschreiten einer vorgegebenen Druckdifferenz zwischen einem Inneren des Strömungsraums und der Umgebung die Einlassöffnung des Strömungsraums zu schließen. Es ist vorteilhaft, wenn Strömungsraum und Widerstandskörper eingerichtet sind, bei Entstehung eines Überdrucks im Inneren des Strömungsraums die Einlassöffnung des Strömungsraums zu schließen. Dadurch kann bei Atempausen und beim Ausatmen verhindert werden, dass Aerosol aus dem Inhalationssystem austritt oder dass der Kompressor Aerosol in den Strömungsraum drückt und diesen kontaminiert, Maße des Widerstandskörpers oder Querschnitte im Strömungsraum verändert oder dass ein durchsichtiger Bereich einer Wand des Strömungsraums beschlägt. Es kann auch verhindert werden, dass ein Anwender durch den Strömungsraum hindurch ausatmet.

Die Einlassöffnung des Strömungsraums ist geschlossen, wenn keine oder nahezu keine Umgebungsluft durch die Einlassöffnung in den Strömungsraum gelangen kann.

In einer Ausführungsform ist die Einlassöffnung mit einer Dichtfläche oder einer Dichtung, vorzugsweise mit einer Gummidichtung versehen. Es ist vorteilhaft, wenn der Widerstandskörper eine Dichtfläche oder eine Dichtung aufweist. Zweckmäßigerweise ist das Inhaliersystem dazu eingerichtet, die Einlassöffnung schnell verschließen zu können. In einer Ausführungsform ist dies dadurch verwirklicht, dass der Widerstandskörper in einem der Einlassöffnung zuwendbaren oder zugewandten Bereich eine plane oder schwach gekrümmte Fläche aufweist. In einer Ausführungsform weist der Widerstandskörper eine Kugelform oder eine Kegelform auf. Dadurch kann der Widerstandskörper die Einlassöffnung besonders einfach und sicher verschließen. Der Widerstandskörper ist zweckmäßigerweise dazu vorgesehen, bei der Ausatmung oder bei Atempausen nach unten zu fallen, die Einlassöffnung zu sperren und damit eine Ventilfunktion auszuführen. Es ist bevorzugt, wenn der Widerstandskörper eingerichtet ist, bei der Ausatmung oder bei Atempausen durch Schwerkrafteinwirkung die Einlassöffnung zu sperren. In einer Ausführungsform ist der Widerstandskörper dazu vorgesehen, bei der Ausatmung oder bei Atempausen unter Einwirkung einer Feder- oder Magnetkraft die Einlassöffnung zu verschließen und somit eine Ventilfunktion zu übernehmen.

Wenn der Flussanzeiger derart eingerichtet ist, dass der Widerstandskörper, vorzugsweise die Kugel die Einlassöffnung bei der Ausatmung nicht vollständig verschließt, kann mit dem Flussanzeiger auf einfache Weise auch ein PEP-System realisiert werden. Bei einem PEP-System liegt ein positiver Ausatemdruck vor. Dieser wird durch einen erhöhten Ausatemwiderstand erzeugt. Es ist besonders bevorzugt, wenn der Flussanzeiger derart eingerichtet ist, dass die Funktion eines PEP-Systems wahlweise zuschaltbar oder abschaltbar ist. Zweckmäßigerweise ist dazu ein variabler Abstandshalter vorgesehen, der vorzugsweise durch einen Schieber in eine PEP-Position bringbar ist, in der er verhindert, dass der Widerstandskörper die Einlassöffnung vollständig verschließen kann.

Es ist zweckmäßig, wenn der Flussanzeiger einen durchsichtigen Bereich einer Wand des Strömungsraums aufweist. So kann auf einfache Weise erreicht werden, dass ein Anwender eine Position des Widerstandskörpers sehen kann. Es ist zweckmäßig, wenn der durchsichtige Bereich in einer Wand des Strömungsraums vorgesehen ist. Ein Bereich des Strömungsraums ist durchsichtig, wenn dessen Lichtdurchlässigkeit so gross ist, dass ein Anwender zumindest erkennen kann, ob sich der Widerstandskörper in dem durchsichtigen Bereich befindet. In einer Ausführungsform sind mehrere durchsichtige Bereiche in der Wand des Strömungsraums vorgesehen. Zweckmäßigerweise kennzeichnet einer der durchsichtigen Bereiche den optimalen Einatemstrom. Bevorzugt ist es, wenn die Lichtdurchlässigkeit so groß ist, dass ein Anwender erkennen kann, wo sich der Widerstandskörper in dem durchsichtigen Bereich befindet. In einer Ausführungsform ist der Strömungsraum vollständig durchsichtig. Es ist vorteilhaft, wenn der Strömungsraum Polypropylen, vorzugsweise transparentes Polypropylen oder Polyamid aufweist.

In einer Ausführungsform ist der Flussanzeiger dazu eingerichtet, eine Position des Widerstandskörpers über ein Magnetsystem auf eine mechanische oder elektronische Anzeige zu übertragen. Zweckmäßigerweise weist das Magnetsystem ein RFID-System auf. In einer Ausführungsform ist der Widerstandskörper mit einem Transponder versehen. Zweckmäßigerweise ist an dem Strömungsraum ein Lesegerät vorgesehen.

Vorzugsweise weist der Flussanzeiger eine Markierung auf. Zweckmäßigerweise ist die Markierung an dem durchsichtigen Bereich vorgesehen. In einer Ausführungsform kennzeichnet die Markierung einen Bereich, in dem sich der Widerstandskörper befindet, wenn ein optimaler Einatemfluss vorliegt. Es ist vorteilhaft, wenn die Markierung verstellbar oder verschiebbar ist. Zweckmäßigerweise ist die Markierung als Klemme oder Klammer ausgeführt. In einer Ausführungsform ist die Markierung als Aufkleber bereitgestellt, der dazu vorgesehen ist, auf das Inhaliersystem geklebt zu werden.

In einer Ausführungsform sind Markierungen spezifischer Flussanzeiger gemäß den Farben entsprechender Düseneinsätze des Verneblers vorzugsweise des PARI LC Sprint kodiert. In einer Ausführungsform ist die Markierung eines Flussanzeigers, der für Kinder vorgesehen ist, gelb wie der gelbe Verneblereinsatz des PARI LC Sprint für Kinder.

Es ist vorteilhaft, wenn die Markierung codierbare Symbole aufweist. So kann einem Symbol eine Bedeutung zugewiesen werden, wie dass ein optimaler Einatemfluss vorliegt, wenn sich der Widerstandskörper im Bereich dieses Symbols befindet. Den Symbolen können je nach erwünschtem Einatemfluss verschiedene Bedeutungen zugewiesen werden. In einer Ausführungsform weist die Skala die Ampelfarben, grün, gelb und rot auf. Es ist vorteilhaft, wenn die Skalen auswechselbar sind, so dass unterschiedliche Skalen eingesetzt werden können. Vorzugsweise sind die Skalen mit einem Clip anclipsbar oder als Etikett aufklebbar. So können unterschiedliche Skalen, für unterschiedliche Anwender wie für Kinder oder Erwachsene vorgesehen werden.

Es ist vorteilhaft, wenn die Markierung oder Skala eingerichtet ist, anzuzeigen, wo im respiratorischen Trakt voraussichtlich die Deposition stattfinden wird. Hohe Flüsse deponieren eher in den Oberen Atemwegen, Bronchien und größeren Bronchiolen, während bei niedrigen Flüssen leichter auch die Peripherie der Lunge und die Alveolen erreicht werden. Die Markierung oder Skala verwendet vorzugsweise Zahlen, Farben oder Rauhigkeiten, zweckmäßigerweise erzeugt mit Hilfe einer Erodierstruktur, zur Kennzeichnung. In einer Ausführungsform sind Markierungen oder Kennzeichnungen erhaben oder vertieft, als Relief ausgebildet, gedruckt oder in einer anderen Farbe gespritzt. Besonders bevorzugt sind Markierungen oder Kennzeichnungen mittels Mehrkomponentenspritzverfahren in einer anderen Farbe gespritzt als der Bereich darum herum.

In einer Ausführungsform ist die Markierung als Skala ausgebildet. Die Skala weist zweckmäßigerweise Striche auf. Vorzugsweise ist die Skala verschiebbar ausgebildet. Wenn die Inhaliervorrichung ein Düsenvernebler ist und die Skala Werte für das Volumen pro Zeit des durchströmenden Gases angibt, ist vorzugsweise berücksichtigt, dass dem Patienten beim Inhalieren zusätzlich Gas aus der Aerosolerzeugungsdüse zugeführt wird. Zweckmäßigerweise wird der Aerosolerzeugungsdüse ein Kompressorstrom von 2 bis 6 l/min zugeführt. Bevorzugt beträgt der Kompressorstrom 4 l/min. In diesem Fall werden dem Patienten 4 l/min zugeführt, wenn sich der Widerstandskörper in seiner untersten Position befindet. Daher ist es zweckmäßig, wenn die Skala dann mit 4 l/min beginnt.

Zweckmäßigerweise ist die Markierung als Orientierungshilfe für einen anzustrebenden Einatemfluss eingerichtet. Die Markierung weist zweckmäßigerweise einen anzustrebenden Bereich auf. In einer Ausführungsform ist die Markierung dazu vorgesehen, zusammen mit einem Experten an dem Inhaliersystem angebracht zu werden. Sinnvollerweise inhaliert der Patient mit einem möglichst idealen Einatemfluss, bevor die Markierung an dem Inhaliersystem angebracht wird. Der Experte kann dem Patienten helfen, den idealen Einatemfluss zu erreichen. Die Markierung kann dann so an dem Inhaliersystem angebracht werden, dass der an der Markierung vorgesehene anzustrebende Bereich den idealen Einatemfluss trifft.

Die willentliche Steuerung des Atemflusses ist nicht ganz trivial. Bei den ersten Versuchen hüpft die Kugel teilweise recht stark. Es ist für viele Personen einfacher, wenn der Einatemwiderstand etwas höher ist und das Zwerchfell hiergegen arbeiten kann. Um dies zu erreichen ist vorzugsweise eine Querschnittsverengung an einer Stelle des Luftweges vorgesehen. Es ist besonders bevorzugt, wenn die Querschnittsverengengung derart beeinflussbar ist, dass unterschiedliche Querschnitte einstellbar sind. In einer Ausführungsform ist die Querschnittsverengung direkt am Einlass angeordnet. Vorzugsweise ist die Querschnittsverengung in Strömungsrichtung nach der Kugel vorgesehen.

Es ist besonders zweckmäßig, wenn die Querschnittsverengung oder Drosselung als Schieber, Klappe, elastisch verformbares Element oder als Bypass-Öffnung ähnlich einem Flötenloch ausgestaltet ist. Letztgenannte ist vorzugsweise in Strömungsrichtung vor der Kugel positioniert. Eine Anpassung über ein Klebetikett auf dem Flötenloch, das nach einer Trainingsphase entfernt werden kann, ist ebenfalls vorteilhaft. In einer Ausführungsform ist aus entformungstechnischen Gründen in dem Bereich, in dem die Strömungsrichtung ähnlich wie in einem Siphon geändert wird, ein Deckel eingesetzt. In einer Ausführungsform ist dieser drehbar ausgestaltet und engt je nach Stellung den Luftkanal mehr oder weniger ein.

Vorteilhaft ist es, wenn die Drosselung ein drehbares Element aufweist, durch das ein Strömungsquerschnitt beeinflussbar ist. Zweckmäßigerweise lässt sich das drehbare Element zumindest teilweise in den Strömungsquerschnitt hinein und wieder herausdrehen.

In einer Ausführungsform ist der Flussmesser in einer Ebene der Hauptachsen zweier Strömungskanäle geteilt. Die beiden Hälften sind vorzugsweise verschweißt oder verclipst. In einer Ausführungsform sind beide Hälften über ein Filmscharnier verbunden.

In einer Ausführungsform weist der Flussmesser ein Auslassventil auf und an der Inhaliervorrichtung, insbesondere an einem Mundstück der Inhaliervorrichtung ist kein Auslassventil vorgesehen.

Es ist vorteilhaft, wenn ein Messverfahren, insbesondere ein kapazitives oder ein auf Magnetfeldänderung basierendes, zur Detektion des Widerstandskörpers, vorzugsweise der Kugel, vorgesehen ist. Vorzugsweise ist der Flussmesser dazu eingerichtet, während der Inhalation derart gefilmt zu werden, dass die Position des Widerstandskörpers erfasst werden kann. Es ist vorteilhaft, wenn der Flussmesser dazu eingerichtet ist, während der Inhalation mit einem Smartphone derart gefilmt zu werden, dass die Position des Widerstandskörpers erfasst werden kann. Es ist besonders bevorzugt, wenn die Bauteile einen starken Kontrast aufweisen, so dass die Bildqualität insbesondere die Schärfe nicht hoch sein muss. Vorzugsweise ist das Smartphone eingerichtet, aus den Bilddaten die Position des Widerstandskörpers zu errechnen und dies für eine verbesserte Darstellung und Rückkopplung auf dem Smartphone-Display darzustellen. Es ist vorteilhaft, wenn das Smartphone eingerichtet ist, die Daten zu speichern. Vorteilhafterweise ist das Smartphone eingerichtet, Daten an einen Empfänger zu übergeben. In einer Ausführungsform ist das Smartphone eingerichtet, Daten eines Sensors, vorzugsweise eines kapazitiven oder magnetischen Sensors zu empfangen. Es ist zweckmäßig, wenn vorgesehen ist, die Übergabe oder das Empfangen von Daten mittels drahtloser Kommunikation, vorzugsweise BlueTooth oder Wifi vorzunehmen. In einer Ausführungsform ist das Smartphone eingerichtet auf den Ort der Deposition und die Menge des deponierten Medikaments rückzuschließen.

Im Folgenden wird die Erfindung anhand von Ausführungsbeispielen unter Bezugnahme auf die beigefügten Figuren genauer beschrieben.
Figur 1 zeigt eine Inhaliervorrichtung aus dem Stand der Technik,
Figur 2 zeigt ein Inhaliersystem mit einer Inhaliervorrichtung und einem Flussmesser,
Figur 3 zeigt ein Inhaliersystem mit einer Inhaliervorrichtung und einem Flussmesser, bei dem Widerstandskörper und Strömungsraum eingerichtet sind, bei Überschreiten einer Grenzströmung einen Durchströmungsquerschnitt in dem Strömungsraum zu verkleinern,
Figur 4 zeigt ein Inhaliersystem mit einer Inhaliervorrichtung und einem Flussmesser, bei dem die Inhaliervorrichtung auch ohne Flussmesser einsetzbar ist,
Figur 5 zeigt ein weiteres Inhaliersystem mit einer Inhaliervorrichtung und einem Flussmesser, bei dem die Inhaliervorrichtung auch ohne Flussmesser einsetzbar ist,
Figur 6 zeigt ein Inhaliersystem, bei dem der Flussmesser in die Inhaliervorrichtung integriert ist,
Figur 7 zeigt eine Inhalierhilfe mit einem Flussmesser,
Figur 8 zeigt eine Inhalierhilfe mit einem Flussmesser, bei der der Flussmesser im Bypass angeordnet ist,
Figur 9 zeigt eine Inhalierhilfe mit einem Flussmesser, bei der der Inhalator mit einer Kappe versehen ist,
Figur 10 zeigt eine Inhalierhilfe mit einem Flussmesser, bei der ein Flussmesser in einer Aufnahme positioniert ist, die auch einen Inhalator aufnehmen kann,
Figur 11 zeigt einen Strömungsraumbehälter mit Führungsrippen,
Figur 12 zeigt einen Strömungsraumbehälter mit einer Führungskontur,
Die Figuren 13 bis 15 zeigen verschiedene Widerstandskörper,
Figur 16 zeigt einen Flussmesser mit einer Skala,
Figur 17 zeigt einen Flussmesser mit unterschiedlichen Skalen,
Figur 18 zeigt einen Flussmesser mit einer speziellen Skala,
Figur 19 zeigt einen Flussmesser mit einer weiteren speziellen Skala,
Figur 20 zeigt ein Inhaliersystem mit codierten Flussmessern,
Die Figuren 21 bis 23 zeigen Flussmesser mit verschiedenen Widerstandskörpern und Strömungsraumbehältern,
Figur 24 zeigt einen Flussmesser mit einem Strömungsraumbehälter, der einen nichtlinearen Querschnitt aufweist,
Figur 25 zeigt einen Flussmesser mit einem Strömungsraumbehälter, der einen gestuften Querschnitt aufweist,
Figur 26 zeigt einen Flussmesser mit einer Bypass-Öffnung,
Figur 27 zeigt einen Flussmesser mit einer Drosselung, die stromaufwärts des Widerstandskörpers vorgesehen ist,
Figur 28 zeigt einen Flussmesser mit einer Drosselung, die stromabwärts des Widerstandskörpers vorgesehen ist,
Figur 29 zeigt einen Flussmesser mit einem elastisch verformbaren Element,
Figur 30 zeigt einen Flussmesser mit einem Steg im Strömungsraumbehälter stromabwärts der Kugel,
Figur 31 zeigt einen Flussmesser mit einer Vorrichtung für positive expiratory pressure,
Figur 32 zeigt einen Flussmesser mit separatem Ausatemventil und
Figur 33 zeigt ein Inhaliersystem, dessen Widerstandskörper mit einem Smartphone detektierbar ist.

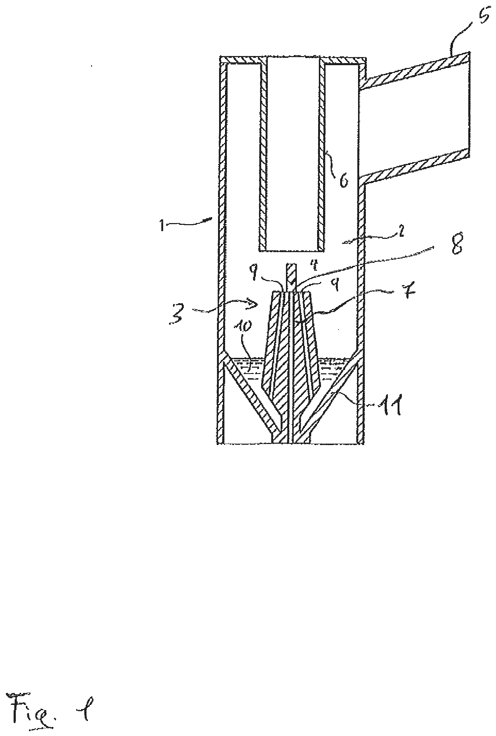

Figur 1 zeigt eine Inhaliervorrichtung aus dem Stand der Technik. Die Inhaliervorrichtung ist ein Düsenvernebler 1 mit einer Verneblerkammer 2. In der Verneblerkammer 2 ist ein Aerosolerzeuger 3 vorgesehen, der in der Lage ist ein Aerosol 4 zu erzeugen. An der Verneblerkammer 2 ist ein Anschluss 5 vorgesehen an den zum Beispiel ein in der Figur nicht gezeigtes Mundstück oder eine Maske angeschlossen werden kann. Die Verneblerkammer 2 weist einen Kamin 6 auf, durch den Umgebungsluft in die Verneblerkammer 2 geleitet werden kann.

Der Aerosolerzeuger 3 umfasst ein Düsenelement 7 mit einer Düsenöffnung 8, durch das im vorliegenden Ausführungsbeispiel Druckluft geleitet werden kann. Weiterhin umfasst der Aerosolerzeuger 3 einen oder mehrere Kanäle 9, durch die ein Medikament 10 aus einem Vorratsbehälter 11 in die Nähe der Düsenöffnung 8 geführt werden kann, aus der im Betrieb die durch das Düsenelement 7 geleitete Druckluft austritt. Auf diese Weise entsteht ein Druckluft-Medikamentengemisch, das in die Verneblerkammer 2 abgegeben wird. Wenn ein Patient beispielsweise über ein Mundstück durch den Anschluss 5 einatmet, wird Umgebungsluft durch den Kamin 6 in die Verneblerkammer 2 gesogen. Diese Umgebungsluft führt das erzeugte Aerosol 4 aus der Verneblerkammer 2 zu dem Anschluss 5. Der Patient kann das Aerosol 4 über ein an den Anschluss 5 angeschlossenes in der Figur nicht gezeigtes Mundstück oder über eine Maske inhalieren.

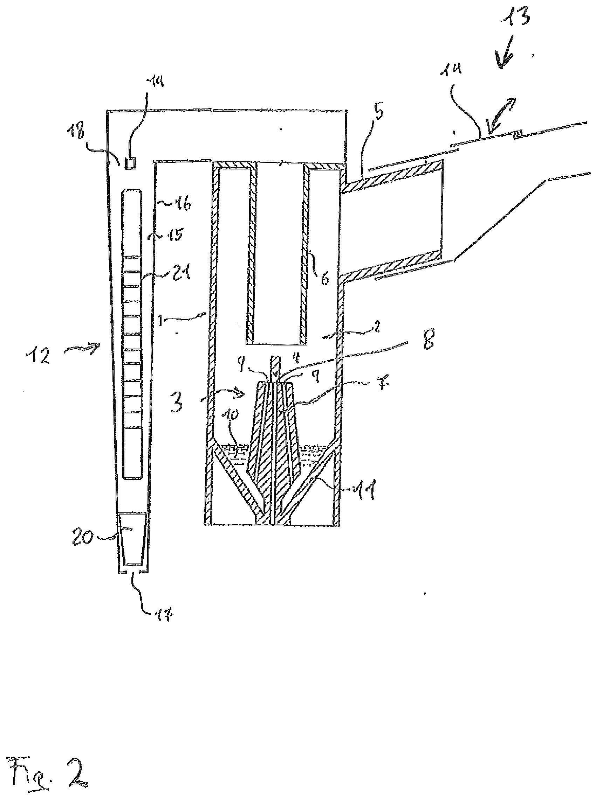

Figur 2 zeigt ein Inhaliersystem mit einer Inhaliervorrichtung und einem Flussmesser 12. Die Inhaliervorrichtung entspricht dem in Figur 1 gezeigten Düsenvernebler 1. An dem Anschluss 5 des Düsenverneblers 1 ist ein Mundstück 13 mit einem Ausatemventil 14 vorgesehen. Der Doppelpfeil deutet die Bewegung des Ausatemventils 14 beim Ein- und Ausatmen an.

Der Flussmesser 12 weist einen konusförmigen Strömungsraum 15 auf, der von einem aus Polypropylen oder Polyamid als Spritzgussteil hergestellten Strömungsraumbehälter 16 begrenzt wird. Der Strömungsraumbehälter 16 weist eine Einlassöffnung 17 und eine Auslassöffnung 18 auf. In der Auslassöffnung 18 ist ein Steg 19 vorgesehen. In dem Strömungsraum 15 ist ein Widerstandskörper 20 vorgesehen, der ebenfalls eine Konusform aufweist. An dem Strömungsraumbehälter 16 ist eine Skala 21 angebracht. Die Skala 21 ist ein bedruckter Aufkleber, auf dem Striche und in der Figur nicht gezeigte Zahlen abgebildet sind, die einen Durchfluss durch die Verneblerkammer 2 angeben.

In Figur 2 ist der Widerstandskörper 20 in einer Position gezeigt, die er bei einem Ausatmen oder einer Atempause eines Patienten einnimmt. Der Widerstandskörper 20 dichtet den Strömungsraum 15 an seinem Umfang ab, so dass keine Umgebungsluft durch den Flussmesser 12 in die Verneblerkammer 2 gelangen kann. Ausatemluft des Patienten kann durch das Ausatemventil 14 in die Umgebung geleitet werden.

Wenn der Patient durch das Mundstück 13 einatmet, schließt sich das Ausatemventil 14. Umgebungsluft wird als Einatemstrom durch die Einlassöffnung 17 in den Strömungsraum 15 geleitet.

Der Widerstandskörper 20 wird durch die entstandenen Druckverhältnisse mit dem Luftstrom in Richtung der Auslassöffnung 18 bewegt, so dass Umgebungsluft zwischen dem Widerstandskörper 20 und dem Strömungsraumbehälter 16 hindurchströmen kann. Je nachdem wie groß der erzeugte Einatemstrom ist, nimmt der Widerstandskörper 20 unterschiedliche Positionen in dem Strömungsraum 15 ein. Dabei begrenzt der Steg 19 den Bereich, in dem sich der Widerstandskörper 20 bewegen kann. Mithilfe der Skala 21 kann abgelesen werden, wie stark der erzeugte Einatemstrom ist, indem der Wert abgelesen wird, an dem der Widerstandskörper 20 sich befindet. Wenn der Einatemstrom sehr hoch ist, wird der Widerstandskörper 20 durch den Steg 19 am Verlassen des Strömungsraums 15 gehindert.

Der Einatemstrom strömt durch die Auslassöffnung 18 hindurch aus dem Strömungsraum 15 heraus und in den Kamin 6 des Düsenverneblers 1. Hier wird der Einatemstrom wie unter Figur 1 beschrieben dem Düsenvernebler 1 zugeführt.

Der Flussmesser 12 kann selbstverständlich auch mit anderen Inhaliervorrichtungen wie beispielsweise mit Meshverneblern verwendet werden.

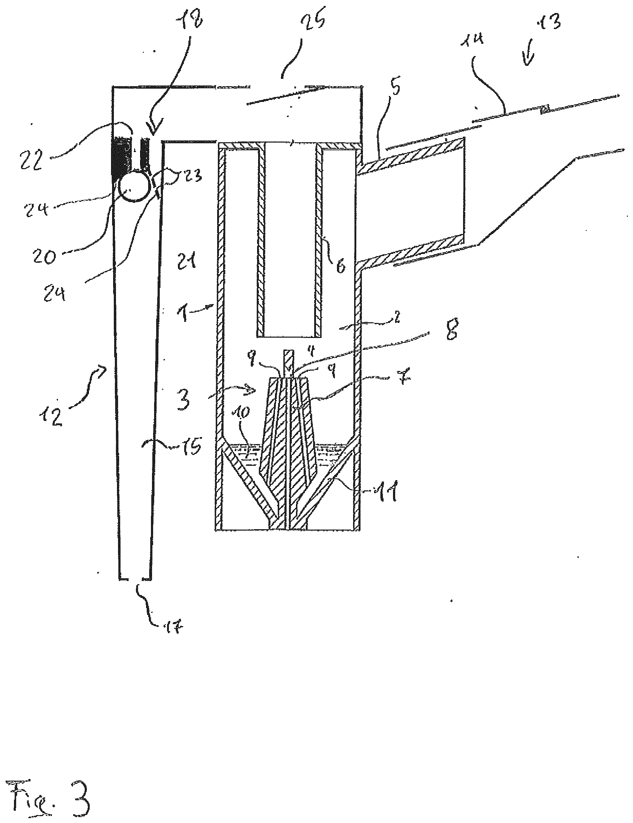

Figur 3 zeigt ein Inhaliersystem mit einer Inhaliervorrichtung und einem Flussmesser 12, bei dem Widerstandskörper 20 und Strömungsraum 15 eingerichtet sind, bei Überschreiten einer Grenzströmung einen Durchströmungsquerschnitt in dem Strömungsraum 15 zu verkleinern.

Das in Figur 3 gezeigte Inhaliersystem entspricht im Wesentlichen, dem in Figur 2 gezeigten Inhaliersystem. Im Folgenden werden die Unterschiede erläutert.

Der Widerstandskörper 20 ist in Figur 3 als Kugel ausgebildet. Die Auslassöffnung 18 weist mehrere Teilöffnungen auf. Es sind eine Hauptöffnung 22 und zwei Nebenöffnungen 23 vorgesehen. Außerdem ist eine Führungseinrichtung 24 für den Widerstandskörper 20 vorgesehen. Die Führungseinrichtung 24 weist im Wesentlichen eine Trichterform auf, die derart auf die Hauptöffnung 22 zuläuft, dass der Widerstandskörper 20 sie bei hohen Einatemströmen verschließt. In der hier gezeigten Ausführungsform sind die Nebenöffnungen 23 in einer Wand der Führungseinrichtung 24 vorgesehen. Diese Wand kann auch als Lochblech oder Sieb ausgestaltet sein oder nur eine Nebenöffnung aufweisen.

Bei hohen Einatemströmen oder Einatemunterdrücken wird der Widerstandskörper 20 in die Führungseinrichtung 24 gezogen und gegen die Hauptöffnung 22 gedrückt, so dass diese verschlossen wird. Einatemluft kann nur noch durch die kleineren Nebenöffnungen 23 strömen. Dadurch steigt der Einatemwiderstand für den Patienten stark an, er kann jedoch noch einatmen. Der Patient erhält so eine Rückmeldung, dass er zu schnell einatmet oder dass der erzeugte Einatemstrom oder der Einatemunterdruck zu groß für eine effektive Inhalation sind. Der Patient kann einen langsameren Einatemstrom erzeugen, so dass der Widerstandskörper 20 sich von der Hauptöffnung 22 weg bewegt und der Strömungsquerschnitt für die Einatemluft wieder größer wird. Dadurch sinkt der Einatemwiderstand.

Es ist auch möglich, keine Nebenöffnungen 23 vorzusehen, so dass mit einem zu hohen Einatemstrom keine Umgebungsluft mehr aus dem Flussmesser 12 in die Verneblerkammer 2 gelangen kann.

Ein weiterer Unterschied zu dem in Figur 2 gezeigten Inhaliersystem ist, dass der Flussmesser 12 hier im Bypass angeordnet ist. Benachbart zu dem Kamin 6 ist ein Einatemventil 25 angeordnet. Einatemluft kann sowohl durch das Einatemventil 25 als auch durch den Flussmesser 12 in die Verneblerkammer 2 geleitet werden.

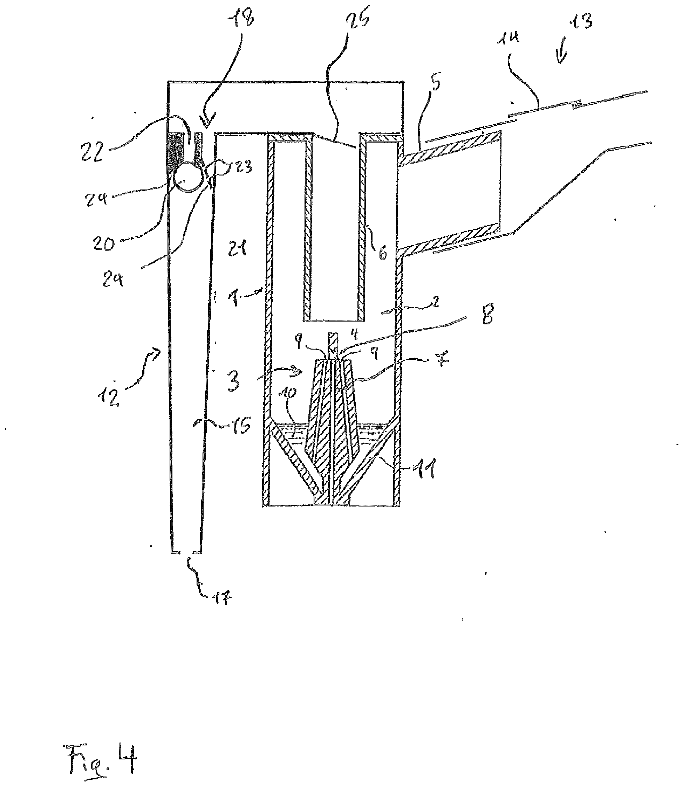

Figur 4 zeigt ein Inhaliersystem mit einer Inhaliervorrichtung und einem Flussmesser 12, bei dem die Inhaliervorrichtung auch ohne Flussmesser 12 einsetzbar ist.

Das in Figur 4 gezeigte Inhaliersystem entspricht im Wesentlichen, dem in Figur 3 gezeigten Inhaliersystem. Im Folgenden werden die Unterschiede erläutert.

Die Inhaliervorrichtung weist ein Einatemventil 25 auf. Der Flussmesser 12 ist lösbar an der Inhaliervorrichtung derart angebracht, dass ein Gas, das durch die Auslassöffnung 18 strömt, zu dem Einatemventil 25 geleitet wird. Die lösbare Verbindung ist eine in Figur 4 nicht gezeigte Steckverbindung.

Durch Lösen der Steckverbindung und Abnehmen des Flussmessers 12 kann die Inhaliervorrichtung auch ohne den Flussmesser 12 betrieben werden.

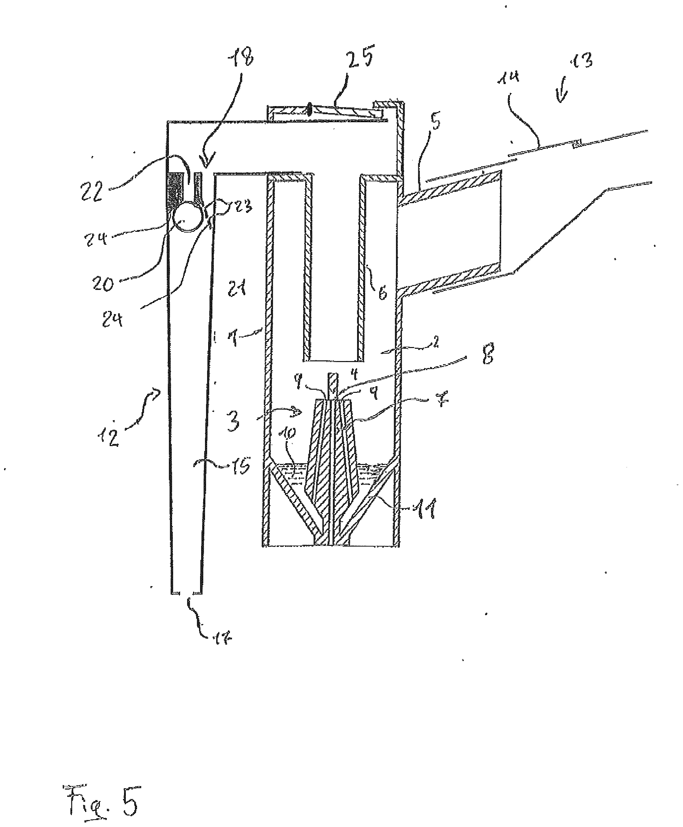

Figur 5 zeigt ein weiteres Inhaliersystem mit einer Inhaliervorrichtung und einem Flussmesser 12, bei dem die Inhaliervorrichtung auch ohne Flussmesser 12 einsetzbar ist. Die Inhaliervorrichtung weist ebenfalls ein Einatemventil 25 auf. Der Flussmesser 12 ist ebenfalls lösbar an der Inhaliervorrichtung angebracht. Der Flussmesser 12 ist jedoch derart an der Inhaliervorrichtung angebracht, dass das Einatemventil 25 blockiert ist. Wenn der Flussmesser 12 von der Inhaliervorrichtung gelöst ist, ist das Einatemventil 25 freigegeben, so dass es öffnen kann. Die lösbare Verbindung von Flussmesser 25 und Inhaliervorrichtung ist in der in Figur 5 gezeigten Ausführungsform durch eine in der Figur nicht dargestellte Steckverbindung gelöst.

Die in Figur 5 gezeigte Inhaliervorrichtung kann ohne den Flussmesser 12 eingesetzt werden. Wenn der Flussmesser 12 mit der Inhaliervorrichtung verbunden ist, kann ein Gasstrom durch die Auslassöffnung 18 in den Kamin 6 geleitet werden ohne ein Einatemventil passieren zu müssen. Ein Patient muss beim Einatmen nicht die Kraft des Einatemventils 25 überwinden.

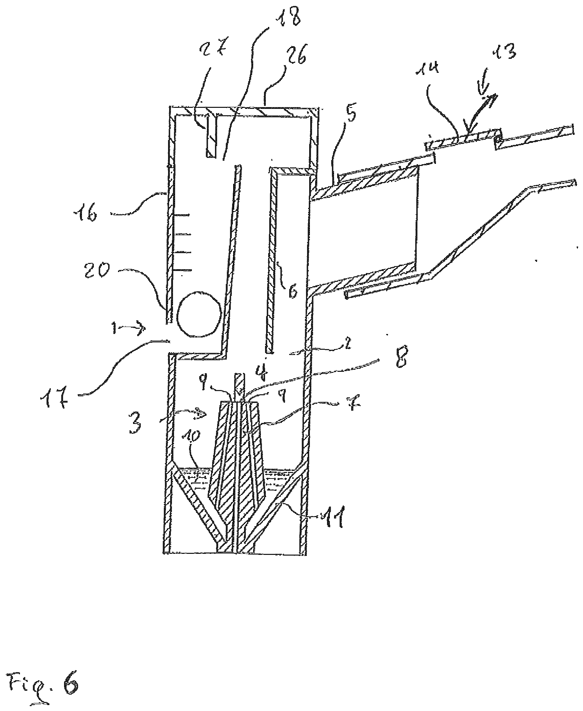

Figur 6 zeigt ein Inhaliersystem, bei dem der Flussmesser 12 in die Inhaliervorrichtung integriert ist.

Der Strömungsraumbehälter 16 ist benachbart zu dem Kamin 6 in das Hauptgehäuse des Düsenverneblers 1 integriert. Die Einlassöffnung 17 ist seitlich an dem Strömungsraumbehälter 16 vorgesehen. Kamin 6 und Strömungsraumbehälter 16 sind von einem Deckel 26 derart abgedeckt, dass ein Luftstrom von der Auslassöffnung 18 des Strömungsraumbehälters 16 durch den Deckel 26 hindurch zu dem Kamin 6 strömen kann.

Ein Deckelstift 27 kann an einer Innenseite des Deckels 26 vorgesehen sein, um zu verhindern, dass der Widerstandskörper 20 bei hohen Einatemflüssen den Strömungsraum verlässt. In einer in Figur 6 nicht gezeigten Ausführungsform sind die Höhe des Deckels 26 und der Strömungsraumbehälter 16 derart ausgestaltet, dass der Widerstandskörper 20 nicht aus dem Strömungsraum heraus gelangen kann.

Das Hauptgehäuse des Düsenverneblers mit Kamin 6 und Strömungsraumbehälter 16 ist in der in Figur 6 gezeigten Ausführung als Spritzgussteil aus Polypropylen ausgeführt.

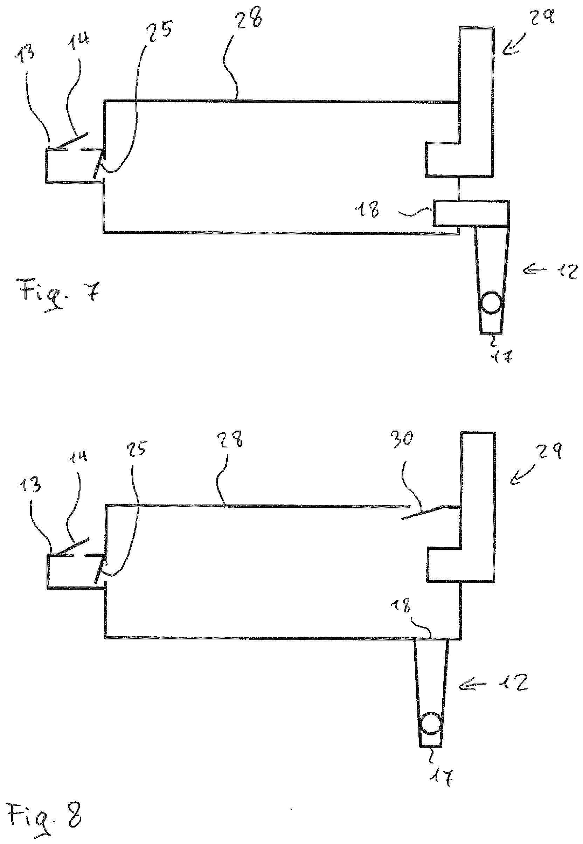

Figur 7 zeigt eine Inhalierhilfe mit einem Flussmesser 12. An einer Aerosolkammer 28 sind einlassseitig ein Inhalator 29 und ein Flussmesser 12 vorgesehen. Dabei sind Inhalator 29 und Flussmesser 12 in dieser Ausführungsform mit einer Rückwand der Aerosolkammer 28 verbunden. Auslassseitig ist ein Einatemventil 25 vorgesehen, das wirksam mit einem Mundstück 13 verbunden ist, das ein Ausatemventil 14 aufweist.

Der Flussmesser 12 funktioniert entsprechend den Flussmessern 12, die in Zusammenhang mit den Figuren 2 bis 6 beschrieben sind. Zum Inhalieren wird der Inhalator 29 ausgelöst, so dass er ein Medikament in die Aerosolkammer 28 abgibt. Der Patient atmet durch das Mundstück 13 ein, das Ausatemventil 14 schließt sich, das Einatemventil 25 öffnet sich und der Patient saugt sich in der Aerosolkammer 28 befindliches Gas durch das Mundstück, so dass er es inhalieren kann. Gleichzeitig strömt Umgebungsluft durch die Einlassöffnung 17 in den Flussmesser 12 hinein und durch die Auslassöffnung 18 aus dem Flussmesser 12 heraus und in die Aerosolkammer 28 hinein. Wie in Zusammenhang mit den Figuren 2 bis 6 beschrieben, kann die durch den Flussmesser 12 hindurchströmende Gasmenge abgelesen werden. Dem Patienten wird zusätzlich zu der durch den Flussmesser 12 hindurchströmenden Gasmenge eine Gasmenge bereitgestellt, die durch den Inhalator 29 strömt. Außerdem wird dem Patienten die Gasmenge aus dem Inhalator 29 selbst zugeführt.

Figur 8 zeigt eine Inhalierhilfe mit einem Flussmesser 12, bei der der Flussmesser 12 im Bypass angeordnet ist. Ein Unterschied zu der in Figur 7 gezeigten Anordnung besteht darin, dass hier Umgebungsluft nicht nur durch den Flussmesser 12 und den Inhalator 29 in die Aerosolkammer 28 hineinströmen kann, sondern auch durch das Einlassventil 30. Ein weiterer Unterschied ist, dass der Flussmesser 12 nicht an der Rückwand, sondern an einer Seitenwand der Aerosolkammer 28 angeordnet ist.

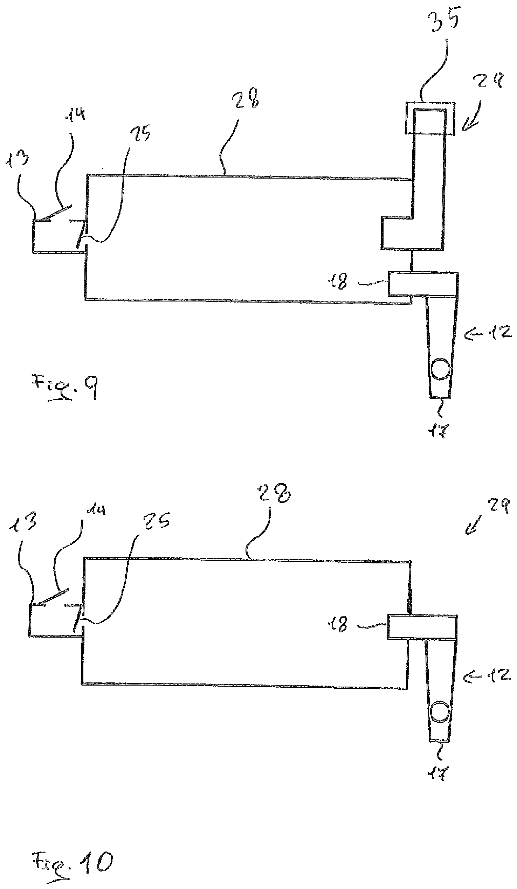

Figur 9 zeigt eine Inhalierhilfe mit einem Flussmesser 12, bei der der Inhalator 29 mit einer Kappe 35 versehen ist, Die Kappe 35 schließt den Inhalator 29 an dessen Einlassbereich luftdicht ab. Dadurch wird vermieden, dass Umgebungsluft durch den Inhalator 29 in die Aerosolkammer 28 gelangen kann. Dadurch kann auf einfache Weise eine genauere Angabe der zugeführten Menge an Umgebungsluft ermöglicht werden. Ungenauigkeiten oder Anpassungen aufgrund unterschiedlich großer Durchlässe für Umgebungsluft bei verschiedenen Inhalatoren 29 können so umgangen werden.

Figur 10 zeigt eine Inhalierhilfe mit einem Flussmesser 12, bei der ein Flussmesser 12 in einer Aufnahme positioniert ist, die auch einen Inhalator 29 aufnehmen kann, Bei Benutzung der Inhalierhilfe wird erst der Inhalator 29 in der Aufnahme angebracht und das Medikament in die Aerosolkammer 28 eingebracht. Dann wird der Inhalator 29 entfernt und der Flussmesser 12 in der Aufnahme angebracht. Auch so können Ungenauigkeiten oder Anpassungen aufgrund unterschiedlich großer Durchlässe für Umgebungsluft bei verschiedenen Inhalatoren 29 umgangen werden.

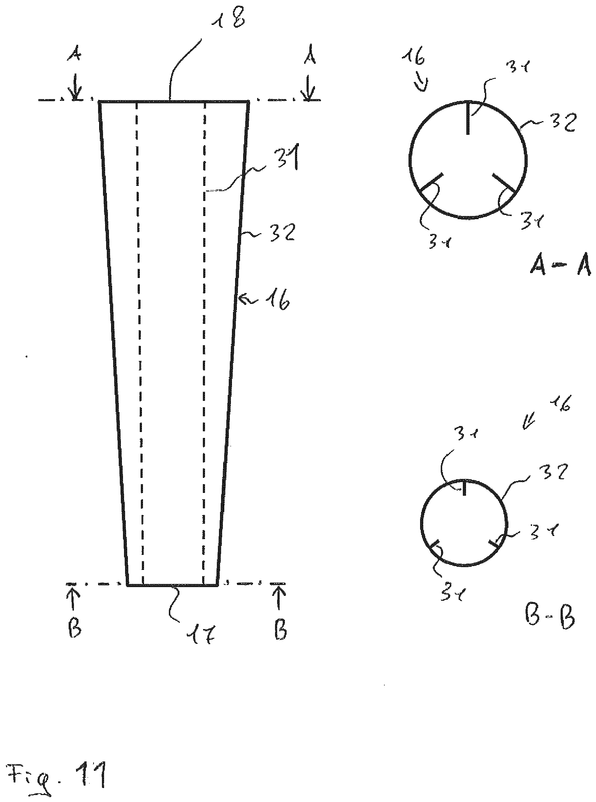

Figur 11 zeigt einen Strömungsraumbehälter 16 mit Führungsrippen 31. Die Außenwand 32 des Strömungsraumbehälters 16 ist kreisförmig ausgestaltet, wobei der Durchmesser des Kreises von der Einlassöffnung 17 zur Auslassöffnung 18 hin kontinuierlich zunimmt. Der Strömungsraumbehälter 16 ist mit drei Führungsrippen 31 versehen, die sich von der Einlassöffnung 17 zur Auslassöffnung 18 erstrecken.

In der in Figur 11 gezeigten Ausführungsform sind die Führungsrippen 31 derart ausgestaltet, dass das zwischen ihnen gebildete Dreieck, über die gesamte Länge gleich groß ist. Dadurch kann ein Widerstandskörper 20 zwischen den Rippen über die gesamte Länge der Rippen gleich gut geführt werden. Es ist möglich, ein Verkanten zu verhindern.

Durch die Verwendung von Führungsrippen 31 kann die Außenwand 32 des Strömungsraumbehälters 16 unabhängig von Führungsfunktionen für den Widerstandskörper 20 gestaltet werden. Sie kann unterschiedliche Querschnitte aufweisen. Es ist jedoch sinnvoll, wenn sie zumindest in dem Bereich, in dem mit Hilfe des Widerstandskörpers 20 ein Gasstrom gemessen werden soll, eine kontinuierlich größer werdende Querschnittsfläche aufweist.

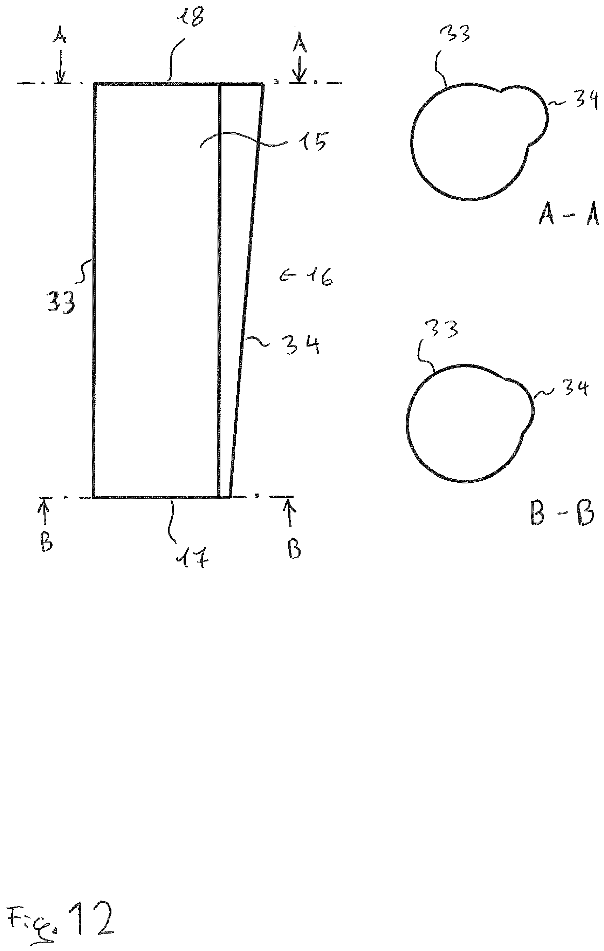

Figur 12 zeigt einen Strömungsraumbehälter 16 mit einer Führungskontur 33. Die Führungskontur 33 erstreckt sich ebenfalls von der Einlassöffnung 17 zur Auslassöffnung 18. Die Führungskontur 33 hat in dieser Ausführungsform im Querschnitt die Kontur eines nicht geschlossenen Kreises. Die Größe der Führungskontur 33 bleibt über die Länge des Strömungsraums 15 gleich. Die Führungskontur 33 ist mit einem Strömungsquerschnitt 34 verbunden. Der Strömungsquerschnitt 34 hat im Querschnitt ebenfalls die Kontur eines nicht geschlossenen Kreises. Die Querschnittsfläche des Strömungsquerschnitts nimmt von der Einlassöffnung 17 zur Auslassöffnung 18 kontinuierlich zu.

In anderen, hier nicht gezeigten Ausführungsformen haben die Querschnitte der Führungskontur 33 und des Strömungsquerschnitts andere Formen, wie beispielsweise dreieckig, quadratisch oder sie weisen unregelmäßige Formen auf. Die Formen der Querschnitte können auch voneinander abweichen.

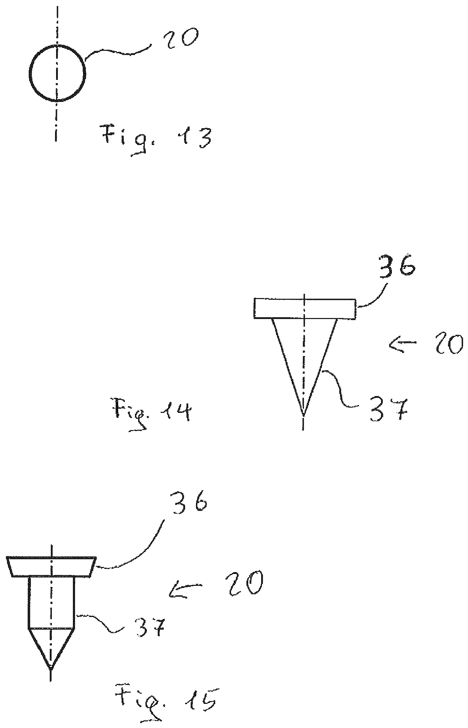

Die Figuren 13 bis 15 zeigen verschiedene Widerstandskörper 20.

Figur 13 zeigt einen kugelförmigen Widerstandskörper 20. Dieser Widerstandskörper 20 ist gut geeignet, um ein Verkanten zu verhindern.

Figur 14 zeigt einen rotationssymmetrischen Widerstandskörper 20. Der Widerstandskörper 20 weist einen zylindrischen Führungsabschnitt 36 und einen kegelförmigen Stabilisierungsabschnitt 37 auf. Der zylindrische Abschnitt ist dazu eingerichtet, an einer Innenseite eines Strömungsraumbehälters 16 anliegen zu können. Der kegelförmige Abschnitt kann den Widerstandskörper 20 in einigen Ausführungsformen des Flussmessers 12 in seiner Lage stabilisieren, indem er ein Kippen des Widerstandskörpers 20 durch sein Gewicht verhindert.

Figur 15 zeigt ebenfalls einen rotationssymmetrischen Widerstandskörper 20. Dieser Widerstandskörper 20 weist ebenfalls einen Führungsabschnitt 36 und einen Stabiliserungsabschnitt 37 auf. Der Führungsabschnitt 36 ist in Form eines Kegelabschnitts ausgestaltet. Der Stabiliserungsabschnitt 37 weist eine zylindrische Form auf, die mit einem Kegel verbunden ist. Auch hier ist der Führungsabschnitt 36 dazu eingerichtet, an einer Innenseite eines Strömungsraumbehälters 16 anliegen zu können. Auch hier kann der Stabilisierungsabschnitt 37 den Widerstandskörper 20 in einigen Ausführungsformen des Flussmessers 12 in seiner Lage stabilisieren, indem er ein Kippen des Widerstandskörpers 20 durch sein Gewicht verhindert.

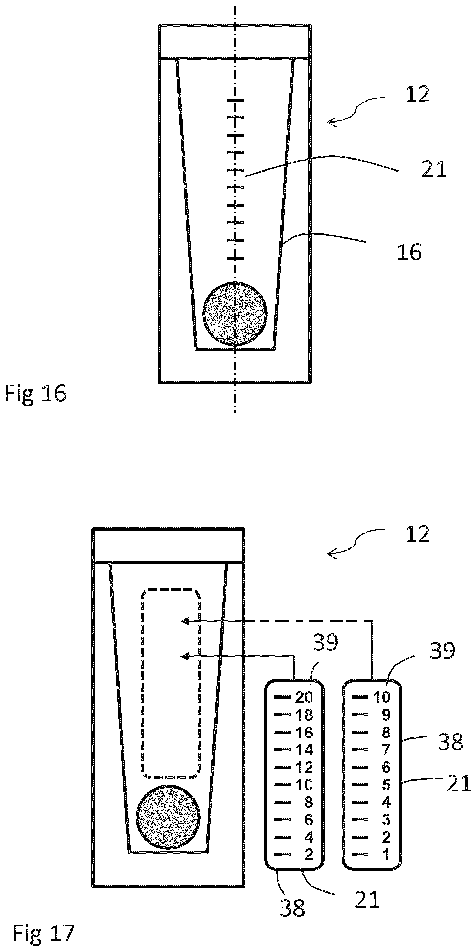

Figur 16 zeigt einen Flussmesser 12 mit einer Skala 21. Der Flussmesser 12 ähnelt dem in Zusammenhang mit Figur 2 beschriebenen Flussmesser 12. Die Skala 21 weist Striche auf, die verschiedene Flüsse kennzeichnen. Die Striche sind durch einen Farbauftrag auf eine Außenseite des Strömungsraumbehälters 16 ausgebildet. In einer nicht gezeigten Ausführungsform weist der Strömungsraumbehälter 16 im Bereich der Striche eine höhere Oberflächenrauhigkeit, erhabene oder vertiefte Strukturen oder ein anderes Material auf als in den Bereichen, die sich benachbart zu den Strichen befinden. In einer nicht gezeigten Ausführungsform ist die Skala im Mehrkomponentenspritzverfahren in einer anderen Farbe gespritzt worden als der Bereich, der benachbart zu der Skala ist.

Figur 17 zeigt einen Flussmesser 12 mit unterschiedlichen Skalen 21. Die Skalen 21 sind mit Skalenbezifferungswerten 39 versehen und als Etikett 38 ausgebildet. Das erste Etikett 38 ist für Erwachsene und das zweite Etikett 38 ist für Kinder vorgesehen. Abhängig davon, ob ein Erwachsener oder ein Kind den Flussmesser 12 verwendet, kann das entsprechende Etikett 38 aufgeklebt werden. Das Etikett 38 für Kinder weist eine feinere Skala 21 mit niedrigeren Skaleneinteilungswerten auf als das Etikett 38 für Erwachsene. Das Etikett 38 für Erwachsene weist einen größeren Messbereich auf als das Etikett 38 für Kinder.

In einer nicht gezeigten Ausführungsform sind die Skalen 21 auswechselbar gestaltet. Sie weisen eine Klemme auf, mit der sie an den Flussmesser 12 angeklemmt werden können.

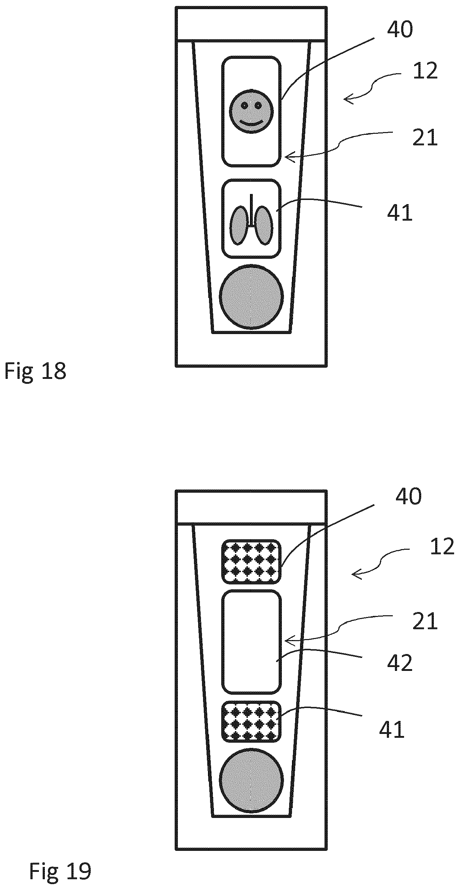

Figur 18 zeigt einen Flussmesser 12 mit einer speziellen Skala 21, die anzeigt, wo im respiratorischen Trakt voraussichtlich die Deposition stattfinden wird. Ein oberer Skalenbereich 40 zeigt ein Symbol, das für einen hohen Fluss steht, der eher in den oberen Atemwegen, Bronchien und größeren Bronchiolen deponiert. Das Symbol ist hier ein Smiley. Ein unterer Skalenbereich 41 zeigt ein Symbol, das für niedrige Flüsse steht, bei denen eher die Peripherie der Lunge erreicht werden kann. Das Symbol ist hier eine Lunge.

Figur 19 zeigt einen Flussmesser 12 mit einer weiteren speziellen Skala 21, die einen oberen Skalenbereich 40, einen unteren Skalenbereich 41 und einen mittleren Skalenbereich 42 aufweist. Der obere und der untere Skalenbereich 40, 41 sind als Struktur oder Relief ausgebildet, so dass sie erhabene und vertiefte Stellen aufweisen. Der mittlere Skalenbereich 42 weist eine polierte Oberfläche auf, so dass der Widerstandskörper 20 in diesem Bereich besonders gut sichtbar ist.

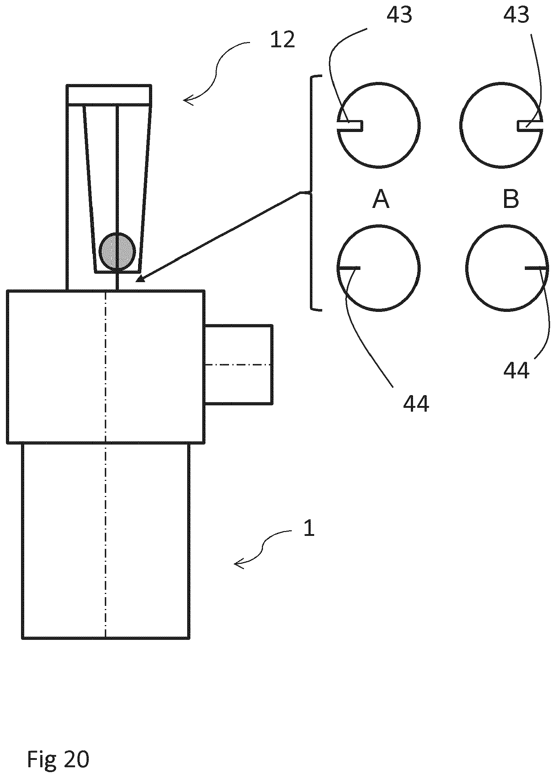

Figur 20 zeigt ein Inhaliersystem mit codiertem Flussmesser 12 und codierter Inhaliervorrichtung 1. Der Flussmesser 12 weist eine Nut 43 auf. Die Inhaliervorrichtung 1 weist eine Rippe 44 auf. Es gibt verschiedene Typen von Flussmessern 12 und Inhaliervorrichtungen 1. Dabei passt nicht jeder Flussmesser 12 zu jeder Inhaliervorrichtung 1. Die Nuten 43 und Rippen 44 sind jeweils derart angeordnet, dass sich ein Flussmesser 12 nur sinnvoll mit einer passenden Inhaliervorrichtung verbinden lässt. In der hier gezeigten Ausführungsform lassen sich zwar alle Flussmesser 12 mit allen Inhaliervorrichtungen 1 verbinden, jedoch lässt sich ein Flussmesser 12 nur verkehrt herum, so dass er nicht ablesbar ist, mit einer nicht passenden Inhaliervorrichtung 1 verbinden.

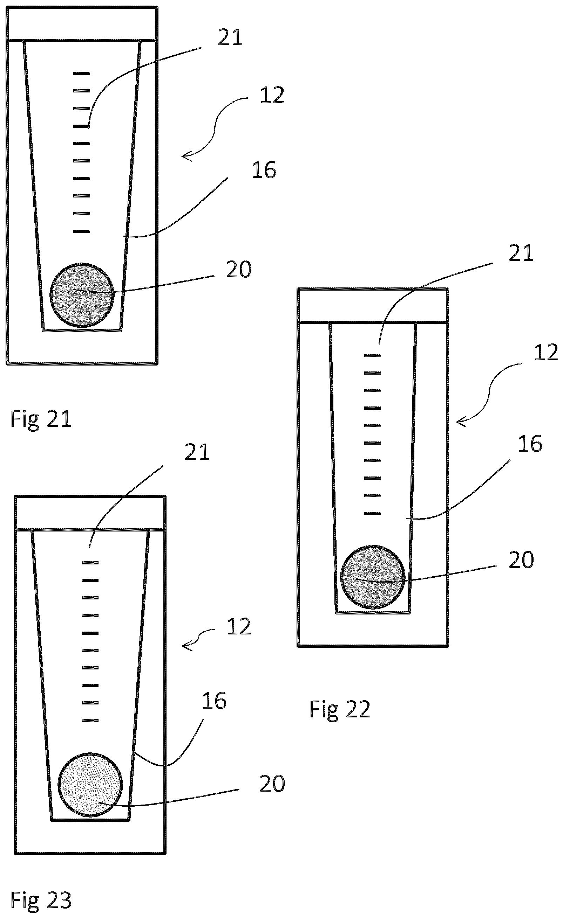

Die Figuren 21 bis 23 zeigen Flussmesser 12 mit verschiedenen Widerstandskörpern 20 und Strömungsraumbehältern 16. Figur 21 zeigt einen Flussmesser 12 für einen Erwachsenen mit einer schweren Kugel 20 als Widerstandskörper 20 und einem konischen Strömungsraumbehälter 16. Auf einer Außenwand des Strömungsraumbehälters 16 ist eine Skala 21 vorgesehen. Die Striche der Skala 21 sind durch Schleifen der jeweiligen Oberflächenbereiche, so dass die Striche eine höhere Oberflächenrauhigkeit aufweisen als die Umgebung, auf den Strömungsraumbehälter 16 aufgebracht worden.

Figur 22 zeigt einen Flussmesser 12 für ein Kind mit einer schweren Kugel 20 als Widerstandskörper 20 und einem konischen Strömungsraumbehälter 16, wobei der Querschnitt des Strömungsraumbehälters 16 in Strömungsrichtung weniger stark zunimmt als bei dem in Figur 21 gezeigten Flussmesser 12. Dadurch, steigt die Kugel 20 bei zunehmender Strömungsgeschwindigkeit schneller in dem Strömungsraumbehälter 16 hoch als bei dem Flussmesser 12 für einen Erwachsenen, der in Figur 21 gezeigt ist. Die Strömungsgeschwindigkeit lässt sich dadurch präziser ablesen. Allerdings lässt sich dadurch nur ein kleinerer Bereich ablesen. Die Kugel ist bei zunehmender Strömungsgeschwindigkeit früher am oberen Ende des Strömungsraumbehälters 16 angelangt als bei dem in Figur 21 gezeigten Flussmesser 12. Eine Skala 21 weist im Vergleich zu der in Figur 21 gezeigten Skala 21 eine feinere Unterteilung und einen kleineren Bereich der messbaren Strömungsgeschwindigkeit auf.

Figur 23 zeigt einen Flussmesser 12 für ein Kind mit einer leichten Kugel 20 und einem konischen Strömungsraumbehälter 16. Auf einer Außenwand des Strömungsraumbehälters 16 ist eine Skala 21 vorgesehen, die an die leichte Kugel 20 angepasst worden ist. Durch das Vorsehen der leichten Kugel 20 als Widerstandskörper 20 steigt die Kugel 20 in dem Strömungsraumbehälter 16 bei niedrigeren Flüssen hoch als die in Figur 21 gezeigte Kugel 20.

Die Farbe der Skala 21 entspricht in dieser Ausführungsform der Farbe eines Verneblereinsatzes oder Düseneinsatzes, der dafür vorgesehen ist, in Verbindung mit dieser Skala 21 eingesetzt zu werden. Die Skala 21 ist gelb. So wird die richtige Kombination von Verneblereinsatz oder Düseneinsatz und Flussmesser 12 erleichtert.

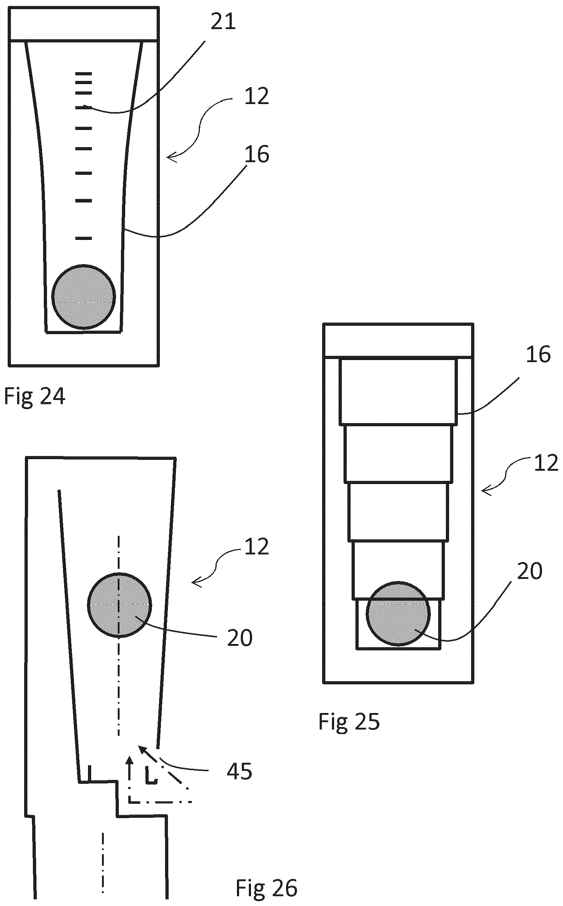

Figur 24 zeigt einen Flussmesser 12 mit einem Strömungsraumbehälter 16, der einen nichtlinearen Querschnitt aufweist. Der Querschnitt ändert sich exponentiell. Die Form des Strömungsraumbehälters 16 ähnelt einem Trompetentrichter. Dadurch ist die Auflösung einer hier vorgesehenen Skala 21 verzerrt, so dass der Bereich, in dem niedrigere Flüsse angezeigt werden, stärker aufgespreizt ist als der Bereich, in dem höhere Flüsse angezeigt werden. So kann der Fluss im Bereich niedrigerer Flüsse insbesondere für Kinder deutlicher angezeigt werden, so dass er leichter abzulesen ist.

Figur 25 zeigt einen Flussmesser 12 mit einem Strömungsraumbehälter 16, der einen gestuften Querschnitt aufweist. Der Strömungsraumbehälter 16 oder Steigrohr weist eine Sequenz von zylindrischen Rohren auf. Dadurch verweilt der hier vorgesehene Widerstandskörper 20 so lange an einem Übergang, bis der Fluss so groß oder klein ist, dass der nächste Zylinder durcheilt wird, bis der Widerstandskörper 20 wieder beim nächsten Übergang verweilt. Durch diese Maßnahme kann die Bewegung des Widerstandskörpers 20 gedämpft werden.

Figur 26 zeigt einen Flussmesser 12 mit einer Bypass-Öffnung 45. Die Bypass-Öffnung 45 ist in Strömungsrichtung vor einem Widerstandskörper 20 positioniert. Diese ist ähnlich wie ein Flötenloch ausgebildet, so dass ein Anwender die Bypass-Öffnung 45 mit einem Finger verschließen kann. Dadurch steigt der Einatemwiderstand. Die willentliche Steuerung des Atemflusses ist nicht ganz trivial. Bei den ersten Versuchen hüpft der Widerstandskörper 20 teilweise recht stark. Für viele Anwender ist einfacher, wenn der Einatemwiderstand etwas höher ist und das Zwerchfell hiergegen arbeiten kann. Dies wird durch das Verschließen der Bypass-Öffnung 45 erreicht. Nach einer Trainingsphase kann auf das Verschließen der Bypass-Öffnung 45 verzichtet werden, so dass kein erhöhter Einatemwiderstand mehr vorliegt. Da bei verschlossener Bypass-Öffnung 45 die Drosselung in Strömungsrichtung vor dem Widerstandskörper 20 erfolgt, verändert der Druckabfall die Strömungsverhältnisse derart, dass die Messung etwas weniger genau ist.

In einer nicht gezeigten Ausführungsform ist ein Klebeetikett vorgesehen, dass auf der Bypass-Öffnung 45 angebracht werden kann und nach der Trainingsphase entfernt werden kann.

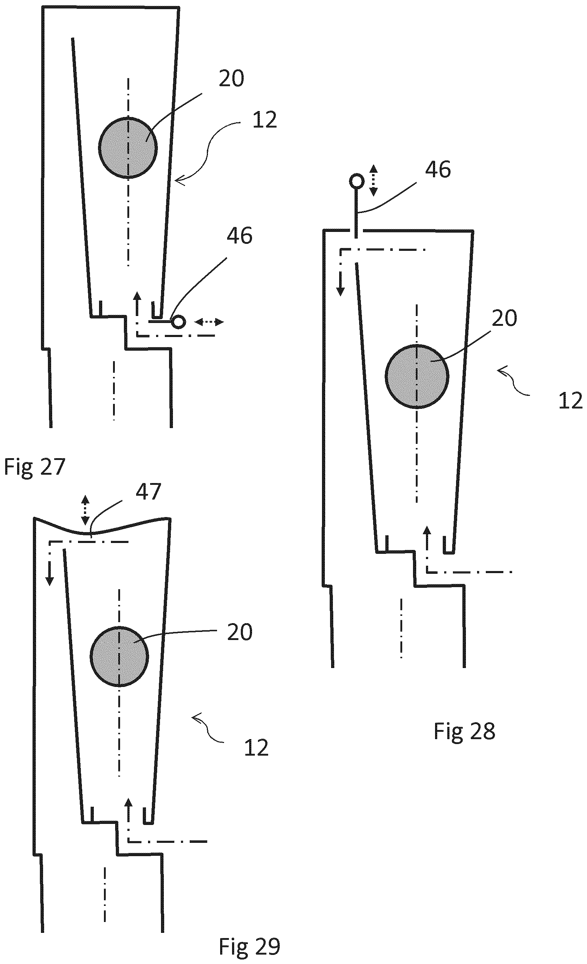

Figur 27 zeigt einen Flussmesser 12 mit einer Drosselung, die stromaufwärts eines Widerstandskörpers 20 vorgesehen ist. Die Drosselung wird durch das Vorsehen eines Schiebers 46 ermöglicht. Durch eine Bewegung des Schiebers 46 in die Strömung hinein kann der Einatemwiderstand erhöht werden, so dass die bei Figur 26 beschriebenen Effekte erzielbar sind. Nach einer Trainingsphase kann der Schieber 46 wieder aus der Strömung heraus gezogen werden.

Figur 28 zeigt einen Flussmesser 12 mit einer Drosselung, die stromabwärts des Widerstandskörpers 20 vorgesehen ist. Die Drosselung wird wie bei der in Figur 27 gezeigten Ausführungsform durch einen Schieber 46 ermöglicht. Da der Schieber 46 stromabwärts des Widerstandskörpers 20 angeordnet ist, werden die Strömungsverhältnisse im Bereich des Widerstandskörpers 20 nicht verändert.

Figur 29 zeigt einen Flussmesser 12 mit einem elastisch verformbaren Element 47, das als Kappe ausgestaltet ist. Durch Druck auf das elastisch verformbare Element 47 kann der Volumenstrom stromabwärts eines Widerstandskörpers 20 gedrosselt werden. Auch dabei können die bei Figur 26 beschriebenen Effekte erzielt werden. Wenn das elastisch verformbare Element 47 nicht nach Innen gedrückt wird, ist der Volumenstrom ungedrosselt.

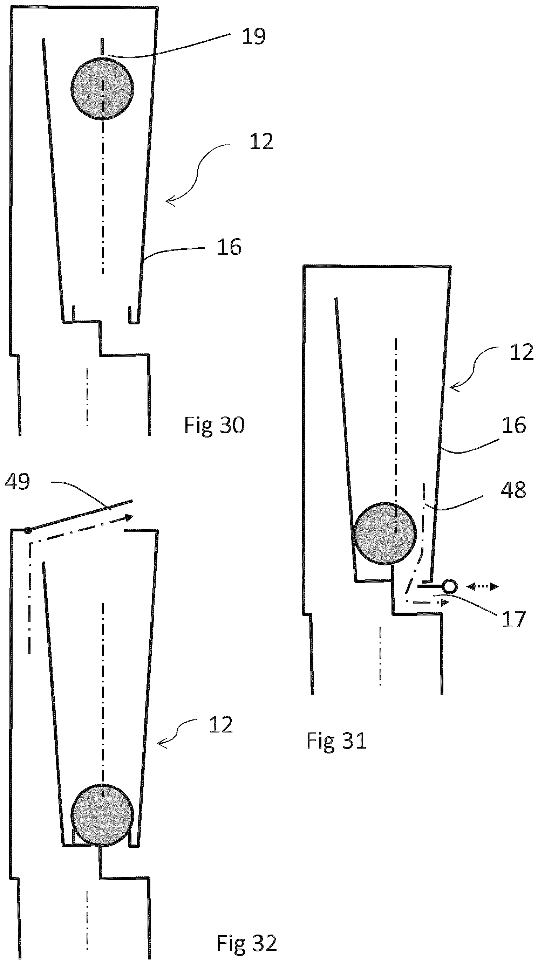

Figur 30 zeigt einen Flussmesser 12 mit einem Steg 19 im Strömungsraumbehälter 16 stromabwärts des Widerstandskörpers 20. Dadurch wird verhindert, dass der Widerstandskörper 20 von einem Anwender verschluckt werden kann.

Figur 31 zeigt einen Flussmesser 12 mit einer Vorrichtung für positive expiratory pressure. In dem Strömungsraumbehälter 16 ist ein Abstandshalter 48 vorgesehen, der derart angeordnet ist, dass der Widerstandskörper 20 die Einlassöffnung 17 nicht vollständig verschließen kann. Dadurch ist es möglich, durch den Flussmesser 12 hindurch auszuatmen. Ein positiver Ausatemdruck, positive expiratory pressure oder PEP entsteht dadurch, dass nur eine kleine Öffnung vorhanden ist und dadurch ein erhöhter Ausatemwiderstand entsteht.

Figur 32 zeigt einen Flussmesser 12 mit separatem Ausatemventil 49. Dadurch ist kein weiteres Ausatemventil 49 im Inhaliersystem erforderlich. Die sich im Stand der Technik in den Mundstücken befindlichen Ausatemventile können entfallen.

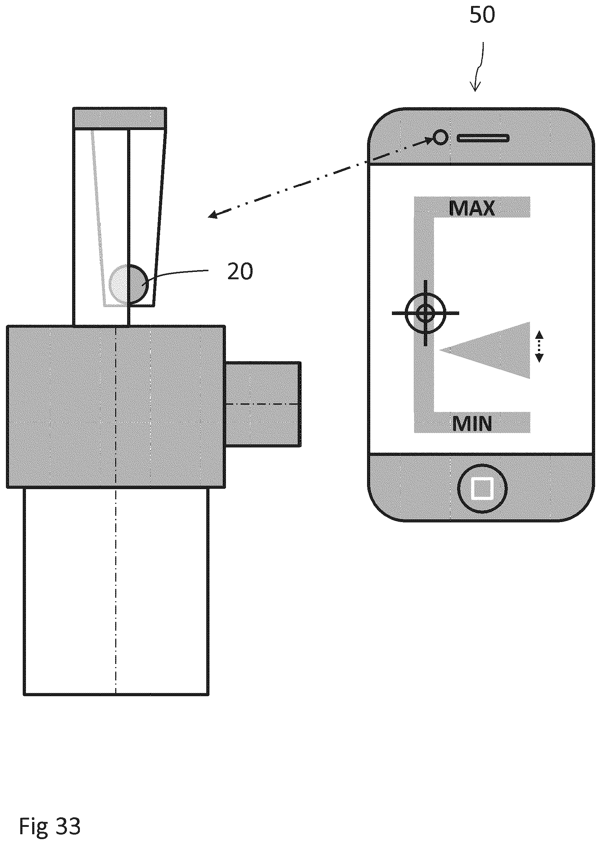

Figur 33 zeigt ein Inhaliersystem, dessen Widerstandskörper 20 mit einem Smartphone 50 detektierbar ist. Der Widerstandskörper 20 ist von außen sichtbar, so dass er während der Inhalation mit einem Smartphone 50 gefilmt werden kann. Das Smartphone 50 ist dazu eingerichtet, die Position des Widerstandskörpers 20 aus den Bilddaten zu errechnen und diese Daten zu speichern. Das Smartphone 50 ist dazu eingerichtet, Rückschlüsse auf den Ort der Deposition und die Menge des deponierten Medikaments zu ziehen.

### Bezugszeichen

- 1: Düsenvernebler
- 2: Verneblerkammer
- 3: Aerosolerzeuger
- 4: Aerosol
- 5: Anschluss
- 6: Kamin
- 7: Düsenelement
- 8: Düsenöffnung
- 9: Kanal
- 10: Medikament
- 11: Vorratsbehälter
- 12: Flussmesser
- 13: Mundstück
- 14: Ausatemventil
- 15: Strömungsraum
- 16: Strömungsraumbehälter
- 17: Einlassöffnung
- 18: Auslassöffnung
- 19: Steg
- 20: Widerstandskörper
- 21: Skala
- 22: Hauptöffnung
- 23: Nebenöffnung
- 24: Führungseinrichtung
- 25: Einatemventil
- 26: Deckel
- 27: Deckelstift
- 28: Aerosolkammer
- 29: Inhalator
- 30: Einlassventil
- 31: Führungsrippe
- 32: Außenwand des Strömungsraumbehälters
- 33: Führungskontur
- 34: Strömungsquerschnitt
- 35: Kappe
- 36: Führungsabschnitt
- 37: Stabilisierungsabschnitt
- 38: Etikett
- 39: Skalenbezifferungswerte
- 40: oberer Skalenbereich
- 41: unterer Skalenbereich
- 42: mittlerer Skalenbereich
- 43: Nut
- 44: Rippe
- 45: Bypass-Öffnung
- 46: Schieber
- 47: elastisch verformbares Element
- 48: Abstandshalter
- 49: Ausatemventil
- 50: Smartphone

### Die folgenden Punkte beschreiben die Erfindung:

1. Inhaliersystem mit
   einer Inhaliervorrichtung und
   einem Flussmesser (12), der einen Strömungsraum (15), einen Widerstandskörper (20) und einen Flussanzeiger (21) aufweist,
   wobei der Strömungsraum (15) eine Einlassöffnung (17), die wirksam mit der Umgebung verbindbar ist, eine Auslassöffnung (18), die wirksam mit einem Innenraum der Inhaliervorrichtung verbindbar ist, und eine Flusswiderstandseinrichtung aufweist,
   das Inhaliersystem eingerichtet ist, eine Zuluft durch die Einlassöffnung (17) in den Strömungsraum (15), durch die Auslassöffnung (18) aus dem Strömungsraum (15) heraus und in den Innenraum der Inhaliervorrichtung zu leiten,
   der Widerstandskörper (20) eingerichtet ist, in dem Strömungsraum (15) verschiedene Positionen einnehmen zu können,
   und der Flussanzeiger (21) eingerichtet ist, eine Position des Widerstandskörpers (20) in dem Strömungsraum (15) anzuzeigen.
2. Inhaliersystem nach Punkt 1, dadurch gekennzeichnet, dass das Inhaliersystem eingerichtet ist, eine Strömungsrichtung der Zuluft stromabwärts des Strömungsraums (15) zu ändern.
3. Inhaliersystem nach einem der vorhergehenden Punkte, dadurch gekennzeichnet, dass der Strömungsraum (15) derart ausgestaltet ist, dass der Widerstandskörper (20) in einem Betriebszustand des Inhaliersystems in dem Strömungsraum (15) eingeschlossen ist.
4. Inhaliersystem nach einem der vorhergehenden Punkte, dadurch gekennzeichnet, dass der Widerstandskörper (20) permanent in dem Strömungsraum (15) eingeschlossen ist.
5. Inhaliersystem nach einem der vorhergehenden Punkte, dadurch gekennzeichnet, dass der Flussmesser (12) dazu eingerichtet ist, trennbar mit der Inhaliervorrichtung verbunden zu sein.
6. Inhaliersystem nach einem der vorhergehenden Punkte, dadurch gekennzeichnet, dass der Strömungsraum (15) eine Führungsvorrichtung für den Widerstandskörper (20) aufweist.
7. Inhaliersystem nach einem der vorhergehenden Punkte, dadurch gekennzeichnet, dass Widerstandskörper (20) und Strömungsraum (15) eingerichtet sind, bei Überschreiten einer Grenzströmung einen Durchströmungsquerschnitt in dem Strömungsraum (15) zu verkleinern.
8. Inhaliersystem nach einem der vorhergehenden Punkte, dadurch gekennzeichnet, dass Widerstandskörper (20) und Strömungsraum (15) eingerichtet sind, bei Unterschreiten einer vorgegebenen Druckdifferenz zwischen einem Inneren des Strömungsraums und der Umgebung, die Einlassöffnung (17) des Strömungsraums (15) zu schließen.
9. Inhaliersystem nach einem der vorhergehenden Punkte, dadurch gekennzeichnet, dass der Flussanzeiger (21) einen durchsichtigen Bereich aufweist.
10. Inhaliersystem nach einem der vorhergehenden Punkte, dadurch gekennzeichnet, dass der Flussanzeiger (21) eine Markierung aufweist.

## Patentansprüche

1. Inhaliersystem mit
einer Inhaliervorrichtung und
einem Flussmesser (12), der einen Strömungsraum (15), einen Widerstandskörper (20) und einen Flussanzeiger (21) aufweist,
wobei der Strömungsraum (15) eine Einlassöffnung (17), die wirksam mit der Umgebung verbindbar ist, eine Auslassöffnung (18), die wirksam mit einem Innenraum der Inhaliervorrichtung verbindbar ist, und eine Flusswiderstandseinrichtung aufweist,
das Inhaliersystem eingerichtet ist, eine Zuluft durch die Einlassöffnung (17) in den Strömungsraum (15), durch die Auslassöffnung (18) aus dem Strömungsraum (15) heraus und in den Innenraum der Inhaliervorrichtung zu leiten,
der Widerstandskörper (20) eingerichtet ist, in dem Strömungsraum (15) verschiedene Positionen einnehmen zu können,
und der Flussanzeiger (21) eingerichtet ist, eine Position des Widerstandskörpers (20) in dem Strömungsraum (15) anzuzeigen.

2. Inhaliersystem nach Anspruch 1, **dadurch gekennzeichnet, dass** das Inhalier-system eingerichtet ist, eine Strömungsrichtung der Zuluft stromabwärts des Strömungsraums (15) zu ändern.

3. Inhaliersystem nach einem der vorhergehenden Ansprüche, **dadurch gekennzeichnet, dass** der Strömungsraum (15) derart ausgestaltet ist, dass der Widerstandskörper (20) in einem Betriebszustand des Inhaliersystems in dem Strömungsraum (15) eingeschlossen ist.

4. Inhaliersystem nach einem der vorhergehenden Ansprüche, **dadurch gekennzeichnet, dass** der Widerstandskörper (20) permanent in dem Strömungsraum (15) eingeschlossen ist.

5. Inhaliersystem nach einem der vorhergehenden Ansprüche, dadurch gekenn-zeichnet, dass der Flussmesser (12) dazu eingerichtet ist, trennbar mit der Inhaliervorrichtung verbunden zu sein.

6. Inhaliersystem nach einem der vorhergehenden Ansprüche, **dadurch gekennzeichnet, dass** der Strömungsraum (15) eine Führungsvorrichtung für den Widerstandskörper (20) aufweist.

7. Inhaliersystem nach einem der vorhergehenden Ansprüche, **dadurch gekennzeichnet, dass** Widerstandskörper (20) und Strömungsraum (15) eingerichtet sind, bei Überschreiten einer Grenzströmung einen Durchströmungsquerschnitt in dem Strömungsraum (15) zu verkleinern.

8. Inhaliersystem nach einem der vorhergehenden Ansprüche, **dadurch gekennzeichnet, dass** Widerstandskörper (20) und Strömungsraum (15) eingerichtet sind, bei Unterschreiten einer vorgegebenen Druckdifferenz zwischen einem Inneren des Strömungsraums und der Umgebung, die Einlassöffnung (17) des Strömungsraums (15) zu schließen.

9. Inhaliersystem nach einem der vorhergehenden Ansprüche, **dadurch gekennzeichnet, dass** der Flussanzeiger (21) einen durchsichtigen Bereich aufweist.

10. Inhaliersystem nach einem der vorhergehenden Ansprüche, **dadurch gekennzeichnet, dass** der Flussanzeiger (21) eine Markierung aufweist.
